(19) Europäisches Patentamt
European Patent Office
Office européen des brevets

(11) EP 3 931 348 B1

(12) EUROPEAN PATENT SPECIFICATION

(45) Date of publication and mention of the grant of the patent:
**09.08.2023 Bulletin 2023/32**

(21) Application number: **20705224.2**

(22) Date of filing: **24.02.2020**

(51) International Patent Classification (IPC):
***C12Q 1/68*** *(2018.01)* ***G01N 5/02*** *(2006.01)*
***G01N 33/15*** *(2006.01)*

(52) Cooperative Patent Classification (CPC):
**C12Q 1/68; G01N 5/025;** C07H 21/00

(86) International application number:
**PCT/EP2020/054716**

(87) International publication number:
**WO 2020/173845 (03.09.2020 Gazette 2020/36)**

# (54) OLIGONUCLEOTIDE FORMULATION METHOD

VERFAHREN ZUR HERSTELLUNG VON OLIGONUKLEOTIDEN

PROCÉDÉ DE FORMULATION D'OLIGONUCLÉOTIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2019 EP 19159353**

(43) Date of publication of application:
**05.01.2022 Bulletin 2022/01**

(73) Proprietor: **Roche Innovation Center Copenhagen A/S**
**2970 Hørsholm (DK)**

(72) Inventors:
- **APPELDORFF LARSEN, Inna**
  **2970 Hørsholm (DK)**
- **MELDGAARD, Michael**
  **2970 Hørsholm (DK)**

(74) Representative: **Rodriguez Novoa, Lorena**
**F. Hoffmann-La Roche AG Patent Department**
**Grenzacherstrasse 124**
**4070 Basel (CH)**

(56) References cited:
**WO-A1-2019/023439 US-A1- 2007 009 009**

- **K. DASH ET AL: "DNA quantification via traceable phosphorus measurement through microwave-assisted UV digestion-ion chromatography", ANALYST, vol. 137, no. 3, 12 December 2011 (2011-12-12), pages 668-674, XP055686897, UK ISSN: 0003-2654, DOI: 10.1039/C2AN15883C**
- **ALISON O. NWOKEOJI ET AL: "Accurate Quantification of Nucleic Acids Using Hypochromicity Measurements in Conjunction with UV Spectrophotometry", ANALYTICAL CHEMISTRY, vol. 89, no. 24, 5 December 2017 (2017-12-05), pages 13567-13574, XP055686899, US ISSN: 0003-2700, DOI: 10.1021/acs.analchem.7b04000**
- **ALEX PEVSNER ET AL: "Infrared Spectroscopic Studies of Major Cellular Components. Part II: The Effect of Hydration on the Spectra of Nucleic Acids", APPLIED SPECTROSCOPY., vol. 55, no. 11, 1 November 2001 (2001-11-01), pages 1502-1505, XP055686901, US ISSN: 0003-7028, DOI: 10.1366/0003702011953720**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF INVENTION

The present invention relates to a method for determining the amount of an oligonucleotide present in a powdered form. The method does not require the experimental determination of the extinction coefficient of the oligonucleotide, or the serial dilution and A260 measurement. The method is, for example, applicable to modified oligonucleotides including 2' sugar modified oligonucleotides, phosphorothioate oligonucleotides, reporter labelled oligonucleotides and conjugated oligonucleotides.

### BACKGROUND

There are numerous potential sites for hydrogen bond formation between an oligonucleotide and water molecules, such as the oxygen atoms in the ribose moiety or internucleoside linkages or base moieties, or in other modifications such as conjugate groups.

Vovelle and Goodfellow, Int. J. Biol. Macromol., 1990, Vol. 12, pp 369-373 reports that the hydration level of DNA is dependent upon both base sequence and DNA conformation.

As a result powdered oligonucleotides have a tendency to take up (absorption) or release water (sorption), depending on the surrounding relative humidity, resulting in a weight gain or loss as a function of the relative humidity.

Unless oligonucleotides are kept in a water free atmosphere, e.g. under nitrogen, it is necessary to determine the water content of an oligonucleotide prior to formulation. This is typically done by solubilisation in water and the measurement of oligonucleotide concentration by absorption at 260nm ($A_{260}$), often using a serial dilution procedure: See Handbook of Analysis of Oligonucleotide and Related Products, p286 - 289, which further discloses the use of Karl Fischer analysis to determine the water content of oligonucleotides, and that for later clinical phases (where great precision is required) an experimental determination of the extinction coefficient of an oligonucleotide should be performed through a dilution series, based upon the Beer-Lambert law ($Abs = \varepsilon 260 * l * C$).

Murphy and Trapane, Analytical Biochemistry 1996, Vol 240, pp273-282 reports on a general phosphate analysis method for determining the concentration of nucleic acid oligomers and their analogues based upon oligomer degradation and determination of stoichiometric phosphorus, a method which does not require prior knowledge of the absorption profile for individual nucleoside residues in the nucleic acid oligomer.

US2007/009009 discloses methods for measuring solid state phase and moisture content of solid samples by combining moisture sorption gravimetry with techniques such as near infra red spectroscopy or Raman spectroscopy.

The physiochemical nature of an oligonucleotide is dependent upon the sequence and presence of modifications of the oligonucleotide - oligonucleotides are routinely modified to enhance their properties, e.g. the incorporation of 2' sugar modified modifications is used to enhance binding affinity of oligonucleotides to complementary target sequences, finding utility in probes and primers as well as research reagents and therapeutics. Typically, the presence of an electronegative substituent at the 2' position of the ribose provides an enhanced binding affinity, and may further provide enhanced nuclease resistance (e.g. as seen with 2'-O-methoxyethyl or LNA nucleosides). The addition of an electronegative substituent and the nature of the electronegative substituent is expected to affect the hydration characteristics of a sugar modified oligonucleotide.

Oligonucleotides for use in biological systems or in vivo, including therapeutics typically contain modified internucleoside linkages - the phosphodiester linkage, which comprises two oxygen atoms, may be replaced with a phosphorothioate or methylphosphonate linkages, for example.

The use of conjugate moieties covalently attached to oligonucleotides is well establish, e.g. for analytical oligonucleotides, where a detectable conjugate moiety is attached to an oligonucleotide, for example a fluorophore or biotin, or for enhancing the pharmacological properties of an oligonucleotide. One widely used conjugate for targeting oligonucleotides, such as antisense oligonucleotides or siRNAs, to the liver is N-acetylgalactosamine (GalNAc) - GalNAc moieties are recognized by the asialoglycoprotein receptor on cells, such as hepatocytes, resulting in internalization of the GalNAc/oligonucleotide molecule. Conjugate groups such as GalNAc provide further sites for hydrogen bonding and are therefore expected to alter the hydration levels as compared to a non-conjugated oligonucleotide.

The present inventors have assessed a range of modified oligonucleotides, including various 2'sugar modifications, backbone linkages and conjugate groups, and have developed a method for determining the amount of an oligonucleotide, or oligonucleotide conjugate present in solid sample, based on the molecular weight of the oligonucleotide or oligonucleotide conjugate, the relative humidity of the atmosphere in which the oligonucleotide or oligonucleotide conjugate is present, and the equilibrated weight of the sold form of the oligonucleotide or oligonucleotide conjugate in the atmosphere. Alternatively stated, the methods of the invention may be used to determining the amount of water present in a sample (e.g. powdered or lyophilized sample) of an oligonucleotide or oligonucleotide conjugate, and thereby the methods may be used to calculate the dry weight or molar quantity of the oligonucleotide or oligonucleotide conjugate

present in the sample.

**STATEMENT OF THE INVENTION**

The invention provides for a method for determining the dry weight of an oligonucleotide salt ($m_{compound\ salt}$) present in a hydrated powdered form of an oligonucleotide salt, said method comprising the steps of:

a. Obtaining a solid powdered form of the oligonucleotide compound salt of known molecular weight ($MW_{compound\ salt}$);
b. Expose the solid powdered form of the oligonucleotide compound salt to an atmosphere for a period of at least about 2 hours, wherein the atmosphere has a relative humidity (RH) of between about 10 - about 70%;
c. Measure the weight of the solid powdered form of the oligonucleotide compound salt after said period of at least about 2 hours (W = $m_{compound\ salt}$+ $H_2Q$);
d. Determine the dry weight of the oligonucleotide;

wherein the dry weight ($m_{compound\ salt}$) is determined by the formula:

$$m_{compound\ salt} = \frac{W}{1 + \frac{Y\ x\ MW_{H_2O}\ x\ Z}{MW_{compound\ salt}}}$$

wherein

*W* is the weight of the solid powdered form of the oligonucleotide salt measured in step c;
Y is determined by the equation Y = a*RH+b, wherein a = a value between 0.0545 to 0.0711, *b* = a value between -0.6818 to 1.365, and RH is the relative humidity referred to in step b;
$MW_{H2O}$ is the molecular weight of water;
Z is the number of nucleotides in the oligonucleotide;
$MW_{compound\ salt}$ is the molecular weight of the oligonucleotide salt.

The invention provides for a method for determining the number of moles of an oligonucleotide salt ($n_{compound\ salt}$) present in a hydrated powdered form of an oligonucleotide salt, said method comprising the steps of:

a. Obtaining a solid powdered form of the oligonucleotide compound salt of known molecular weight ($MW_{compound\ salt}$);
b. Expose the solid powdered form of the oligonucleotide compound salt to an atmosphere for a period of at least about 2 hours, wherein the atmosphere has a known relative humidity (RH) of between about 10 - about 70%;
c. Measure the weight of the solid powdered form of the oligonucleotide compound salt after said period of at least about 2 hours (W = $m_{compound\ salt}$+ $H_2O$);
d. Determine the dry weight of the oligonucleotide;

wherein the dry weight ($m_{compound\ salt}$) is determined by the formula:

$$m_{compound\ salt} = \frac{W}{1 + \frac{Y\ x\ MW_{H_2O}\ x\ Z}{MW_{compound\ salt}}}$$

wherein
*W* is the weight of the solid powdered form of the oligonucleotide salt measured in step c;
Y is determined by the equation Y = a*RH+b, wherein a = a value between 0.0545 to 0.0711, *b* = a value between -0.6818 to 1.365, and RH is the relative humidity referred to in step b;
$MW_{H2O}$ is the molecular weight of water;
Z is the number of nucleotides in the oligonucleotide;
$MW_{compound\ salt}$ is the molecular weight of the oligonucleotide salt.

e. Calculate the number of moles of an oligonucleotide salt ($n_{compound\ salt}$) using the formula:

$$n_{compound\ salt} = m_{compound\ salt} / MW_{compound\ salt}$$

The methods of the invention may further comprise the step of aliquoting a known dry weight of the oligonucleotide salt into a vial. The vial maybe a sterile vial.

The invention provides for a method for preparing a solvent formulation of an oligonucleotide salt with known concentration ($m_{compound\ salt}$ /unit volume), said method comprising determining the dry weight of the oligonucleotide salt ($m_{compound\ salt}$) using the method according to the invention, and subsequently dissolving a calculated dry weight of the oligonucleotide salt ($m_{compound\ salt}$) in a known volume of solvent to provide the solvent formulation of an oligonucleotide salt with known concentration ($m_{compound\ salt}$ /unit volume).

The invention provides for a method for preparing a solvent formulation of an oligonucleotide salt with known molar concentration ($n_{compound\ salt}$ /unit volume), said method comprising determining the number of moles of an oligonucleotide salt ($n_{compound\ salt}$) according to the method of the invention, and subsequently dissolving a number of moles of the oligonucleotide salt in a known volume of solvent to provide the solvent formulation of an oligonucleotide salt with known molar concentration $n_{compound\ salt}$ /unit volume).

The method of the invention may comprise a further step of sterilizing the solvent formulation, for example by filter sterilization.

The methods of the invention may further comprise the step of aliquoting the solvent formulation into a vial or syringe. The sterilization step may be performed prior to, during or subsequent to the aliquoting step.

For experimental or in vivo use, for example, the formulation may be sterilized prior to use, e.g. the vial or syringe containing the solvent formulation may be sterile.

After the aliquoting step, wherein the method further comprises the step of aliquoting the solvent formulation into a vial, the method may comprise a further step of removing the solvent from the solvent composition to provide a vial containing a known quantity of dry oligonucleotide salt. The solvent may be removed by, for example, freeze drying, lyophilisation or by precipitation.

In some embodiments, the oligonucleotide present in the oligonucleotide salt comprises a conjugate moiety, *i.e.* the oligonucleotide is in the form of an oligonucleotide conjugate. In some embodiments, the oligonucleotide comprises a carbohydrate conjugate moiety, such as a GalNAc conjugate moiety.

In some embodiments, the atmosphere of step b) has a relative humidity of between about 10 - about 60%, such as a relative humidity of between about 20 - about 50%.

In some embodiments, the period referred to in step b. (Acclimatization period) is at least about 2 hours or at least about 3 hours or at least about 4 hours.

In some embodiments, the oligonucleotide comprises one or more 2' sugar modified nucleosides, such as a 2' sugar modified nucleotide which comprises an electronegative substituent / atom. In some embodiments, the oligonucleotide comprises one or more 2' sugar modified nucleosides independently selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, 2'-F-ANA nucleoside, and a LNA nucleoside(s).

In some embodiments, the oligonucleotide comprises one or more LNA nucleosides.

In some embodiments, the oligonucleotide comprises one or more 2'-O-methoxyethyl nucleosides (2'-MOE).

In some embodiments, the oligonucleotide comprises phosphorothioate internucleoside linkages.

In some embodiments, the oligonucleotide comprises phosphorodithioate internucleoside linkages.

In some embodiments, the oligonucleotide comprises phosphorothioate and phosphorodithioate internucleoside linkages.

In some embodiments, the oligonucleotide comprises both phosphorothioate internucleoside linkages and phosphodiester internucleoside linkages.

In some embodiments, the ionic cation of the salt is selected from a metal cation, such as a sodium or potassium cation, or an ammonium cation.

## FIGURES

**Figure 1:** example of a DVS analysis of an average compound.

**Figure 2:** The graph displays the correlation between RH and Ratio of water molecules per nucleotide for all analyzed compounds. The graph is based on the average values and does not distinguish between results from absorption and sorption during DVS analysis. Ratio of water molecules per nucleotide of the oligonucleotide salts tested (Compounds 1 - 20) between relative humidity range 10 - 90%. Note linearity for all compounds between RH 10% - 60%, and all compounds except the FAM labelled compound (20) between RH 10% - 70%.

**Figure 3:** The figure shows connection between equation 4 and Ratio-model. Building of Ratio-model was done using data from DVS-analysis and equation 4. Change in mass for new compounds can be calculated using Ratio-model followed by equation 4.
**Figure 4:** The graph shows trend lines based on average, maximum and minimum values from the converted DVS analysis. 70 % RH for maximum values is excluded from this model.
**Figure 5:** Comparison of the DVS ration-model method with A260 absorption based method.
**Figure 6:** Detailed analysis of absorption and sorption during DVS analysis.
**Figure 7:** Ratio of water molecules per nucleotide of the oligonucleotide salts tested (Compounds 1 - 20) between relative humidity range 10 - 65% (close-up of Figure 2).

## DEFINITIONS

***Dry weight***

The term dry weight refers to the calculated weight of the compound salt in solid form in the absence of water, *i.e.* without any hydration. For formulation of compound salts at a specific concentration or dosage it is required that the dry weight of the compound salt is known prior to the formulation.

***Formulation***

The term formulation refers to the combination (mixture) of the compound salt with one or more further compounds. In some embodiments the formulation comprises the solubilisation of the compound salt in a suitable solvent, such as in water. In some embodiments, the formulation may be made with sterile reagents or may be sterilized after the formulation step, providing a sterile formulation. Formulation may further comprise the addition of further components than e.g. the compound and solvent, for example a preservative may be added as part of the formulation.

***Oligonucleotide salt***

The term oligonucleotide salt refers to the oligonucleotide compound in the salt form. The term includes salts of oligonucleotide conjugate compounds as well as salts of unconjugated oligonucleotide compounds.
The oligonucleotide salt is advantageously present in a solid powdered form, and may for example be a sample or a batch of an oligonucleotide salt.

***Hydrated form /Hydrated powdered form***

The term hydrated form refers to the compound salt in the presence of water molecules, and encompasses any degree of hydration, including partially hydrated, where there is some hydrogen bond formation between the compound and water molecules, and fully hydrated forms wherein all hydration sites present on the compound form hydrogen bonds with water molecules.
The hydrated form is a solid form.
Hydrated powdered form refers to hydrated solid compounds which are in powder form, such as a powder form prepared by lyophilization, spray drying or precipitation. Powdered forms are advantageously used in the present invention to ensure rapid and even DVS throughout the sample or batch of oligonucleotide salt.

***Molecular weight (MW)***

The molecule weight of the oligonucleotide salt is molecular weight of the compound salt in dry form (*i.e.* in the absence of hydration), and may be the calculated MW based on the known chemical structure of the oligonucleotide compound salt.

***Oligonucleotide***

The term "oligonucleotide" as used herein is defined as it is generally understood by the skilled person as a molecule comprising two or more covalently linked nucleosides. Such covalently bound nucleosides may also be referred to as nucleic acid molecules or oligomers. The oligonucleotide is a synthetic oligonucleotide. Oligonucleotides are commonly made in the laboratory by solid-phase chemical synthesis followed by purification and isolation. When referring to a sequence of the oligonucleotide, reference is made to the sequence or order of nucleobase moieties, or modifications thereof, of the covalently linked nucleotides or nucleosides. The oligonucleotide is man-made, and is chemically synthesized, and is typically purified or isolated. The oligonucleotide may comprise one or more modified nucleosides or nucleotides, such as 2' sugar modified nucleosides. Advantageously, the oligonucleotide is a mixed sequence oligonucleotide that comprises at least 2, such as at least 3 different nucleobases, selected from the group A, T, C, G and U, wherein C may be 5-methyl cytosine.

Advantageously, the oligonucleotide may have a length of 8 - 25 nucleotides, for example in the case of an antisense oligonucleotide or a primer or probe.

In some embodiments, the oligonucleotide may be an antisense oligonucleotide.

In some embodiments, the oligonucleotide is a gapmer oligonucleotide. In some embodiments, the oligonucleotide is a mixmer oligonucleotide.

In some embodiments, the oligonucleotide may comprise a conjugate group.

In some embodiments, the oligonucleotide may be an oligonucleotide probe or a primer. In some embodiments, the oligonucleotide may comprise a reporter group.

In some embodiments, the oligonucleotide may be a strand of an siRNA. In some embodiments, the oligonucleotide may be a ribozyme or an aptamer.

***Nucleotides***

Nucleotides are the building blocks of oligonucleotides and polynucleotides, and for the purposes of the present invention include both naturally occurring and non-naturally occurring nucleotides. In nature, nucleotides, such as DNA and RNA nucleotides comprise a ribose sugar moiety, a nucleobase moiety and one or more phosphate groups (which is absent in nucleosides). Nucleosides and nucleotides may also interchangeably be referred to as "units" or "monomers".

***Modified nucleoside***

The term "modified nucleoside" or "nucleoside modification" as used herein refers to nucleosides modified as compared to the equivalent DNA or RNA nucleoside by the introduction of one or more modifications of the sugar moiety or the (nucleo)base moiety. In a preferred embodiment the modified nucleoside comprise a modified sugar moiety. The term modified nucleoside may also be used herein interchangeably with the term "nucleoside analogue" or modified "units" or modified "monomers". Nucleosides with an unmodified DNA or RNA sugar moiety are termed DNA or RNA nucleosides herein. Nucleosides with modifications in the base region of the DNA or RNA nucleoside are still generally termed DNA or RNA if they allow Watson Crick base pairing.

***Modified internucleoside linkage***

In some embodiments, the oligonucleotide comprises one or more modified internucleoside linkage.

The term "modified internucleoside linkage" is defined as generally understood by the skilled person as linkages other than phosphodiester (PO) linkages, that covalently couples two nucleosides together. The oligonucleotide may therefore comprise modified internucleoside linkages. Modified internucleoside linkages may for example be selected from the group comprising phosphorothioate, diphosphorothioate and boranophosphate. In some embodiments the internucleoside linkage comprises sulphur (S), such as a phosphorothioate internucleoside linkage. Phosphorothioate internucleoside linkages are useful for use in antisense oligonucleotides, particularly for in vivo use.

With the oligonucleotides referred to in the context of the invention, in some embodiments it is advantageous to use phosphorothioate internucleoside linkages. In some embodiments at least 50% of the internucleoside linkages in the oligonucleotide, or contiguous nucleotide sequence thereof, are phosphorothioate, such as at least 60%, such as at least 70%, such as at least 75%, such as at least 80% or such as at least 90% of the internucleoside linkages in the oligonucleotide, or contiguous nucleotide sequence thereof, are phosphorothioate. In some embodiments all of the internucleoside linkages of the oligonucleotide, or contiguous nucleotide sequence thereof, are phosphorothioate. In some embodiments, the oligonucleotide comprises both phosphorothioate internucleoside linkages and at least one phosphodiester linkage, such as 2, 3 or 4 phosphodiester linkages, in addition to the phosphorodithioate linkage(s).

***Nucleobase***

The term nucleobase includes the purine (e.g. adenine and guanine) and pyrimidine (e.g. uracil, thymine and cytosine) moiety present in nucleosides and nucleotides which form hydrogen bonds in nucleic acid hybridization. In the context of the present invention the term nucleobase also encompasses modified nucleobases which may differ from naturally occurring nucleobases, but are functional during nucleic acid hybridization. In this context "nucleobase" refers to both naturally occurring nucleobases such as adenine, guanine, cytosine, thymidine, uracil, xanthine and hypoxanthine, as well as non-naturally occurring variants. Such variants are for example described in Hirao et al (2012) Accounts of

Chemical Research vol 45 page 2055 and Bergstrom (2009) Current Protocols in Nucleic Acid Chemistry Suppl. 37 1.4.1.

In some embodiments the nucleobase moiety is modified by changing the purine or pyrimidine into a modified purine or pyrimidine, such as substituted purine or substituted pyrimidine, such as a nucleobased selected from isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine and 2-chloro-6-aminopurine.

The nucleobase moieties may be indicated by the letter code for each corresponding nucleobase, e.g. A, T, G, C or U, wherein each letter may optionally include modified nucleobases of equivalent function. For example, in the exemplified oligonucleotides, the nucleobase moieties are selected from A, T, G, C, and 5-methyl cytosine. Optionally, for LNA gapmers, 5-methyl cytosine LNA nucleosides may be used.

***Modified oligonucleotide***

The term modified oligonucleotide describes an oligonucleotide comprising one or more sugar-modified nucleosides and/or modified internucleoside linkages. The term chimeric" oligonucleotide is a term that has been used in the literature to describe oligonucleotides with modified nucleosides.

***High affinity modified nucleosides***

A high affinity modified nucleoside is a modified nucleotide which, when incorporated into the oligonucleotide enhances the affinity of the oligonucleotide for its complementary target, for example as measured by the melting temperature ($T^m$). A high affinity modified nucleoside preferably result in an increase in melting temperature between +0.5 to +12°C, more preferably between +1.5 to +10°C and most preferably between+3 to +8°C per modified nucleoside. Numerous high affinity modified nucleosides are known in the art and include for example, many 2' substituted nucleosides as well as locked nucleic acids (LNA) (see e.g. Freier & Altmann; Nucl. Acid Res., 1997, 25, 4429-4443 and Uhlmann; Curr. Opinion in Drug Development, 2000, 3(2), 293-213).

***Sugar modifications***

The oligomer may comprise one or more nucleosides which have a modified sugar moiety, *i.e.* a modification of the sugar moiety when compared to the ribose sugar moiety found in DNA and RNA.

Numerous nucleosides with modification of the ribose sugar moiety have been made, primarily with the aim of improving certain properties of oligonucleotides, such as affinity and/or nuclease resistance.

Such modifications include those where the ribose ring structure is modified, *e.g.* by replacement with a hexose ring (HNA), or a bicyclic ring, which typically have a biradicle bridge between the C2 and C4 carbons on the ribose ring (LNA), or an unlinked ribose ring which typically lacks a bond between the C2 and C3 carbons (e.g. UNA). Other sugar modified nucleosides include, for example, bicyclohexose nucleic acids (WO2011/017521) or tricyclic nucleic acids (WO2013/154798). Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in DNA and RNA nucleosides. Substituents may, for example be introduced at the 2', 3', 4' or 5' positions.

***2' sugar modified nucleosides***

A 2' sugar modified nucleoside is a nucleoside which has a substituent other than H or -OH at the 2' position (2' substituted nucleoside) or comprises a 2' linked biradicle capable of forming a bridge between the 2' carbon and a second carbon in the ribose ring, such as LNA (2' - 4' biradicle bridged) nucleosides.

Indeed, much focus has been spent on developing 2' sugar substituted nucleosides, and numerous 2' substituted nucleosides have been found to have beneficial properties when incorporated into oligonucleotides. For example, the 2' modified sugar may provide enhanced binding affinity and/or increased nuclease resistance to the oligonucleotide. Examples of 2' substituted modified nucleosides are 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleoside. For further examples, please see e.g. Freier & Altmann; Nucl. Acid Res., 1997, 25, 4429-4443 and Uhlmann; Curr. Opinion in Drug Development, 2000, 3(2), 293-213, and Deleavey and Damha, Chemistry and Biology 2012, 19, 937. Below are illustrations of some 2' substituted modified nucleosides.

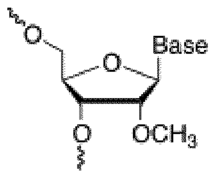
2'-*O*-Me

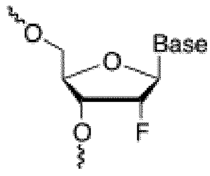
2'F-RNA

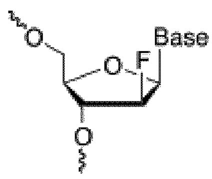
2'F-ANA

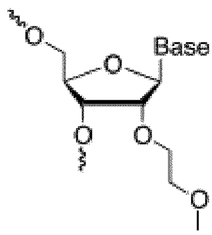
2'-*O*-MOE

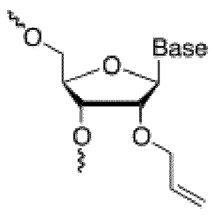
2'-*O*-Allyl

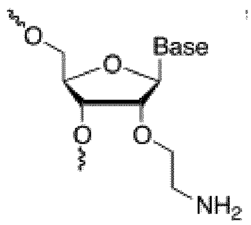
2'-*O*-Ethylamine

In relation to the present invention 2' substituted sugar modified nucleosides does not include 2' bridged nucleosides like LNA.

***Locked Nucleic Acid Nucleosides (LNA nucleoside)***

A "LNA nucleoside" is a 2'- modified nucleoside which comprises a biradical linking the C2' and C4' of the ribose sugar ring of said nucleoside (also referred to as a "2'- 4' bridge"), which restricts or locks the conformation of the ribose ring. These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into an oligonucleotide for a complementary RNA or DNA molecule. This can be routinely determined by measuring the melting temperature of the oligonucleotide/complement duplex.

Non limiting, exemplary LNA nucleosides are disclosed in WO 99/014226, WO 00/66604, WO 98/039352 , WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett. 12, 73-76, Seth et al. J. Org. Chem. 2010, Vol 75(5) pp. 1569-81, and Mitsuoka et al., Nucleic Acids Research 2009, 37(4), 1225-1238, and Wan and Seth, J. Medical Chemistry 2016, 59, 9645-9667.

Further non limiting, exemplary LNA nucleosides are disclosed in Scheme 1.

Scheme 1:

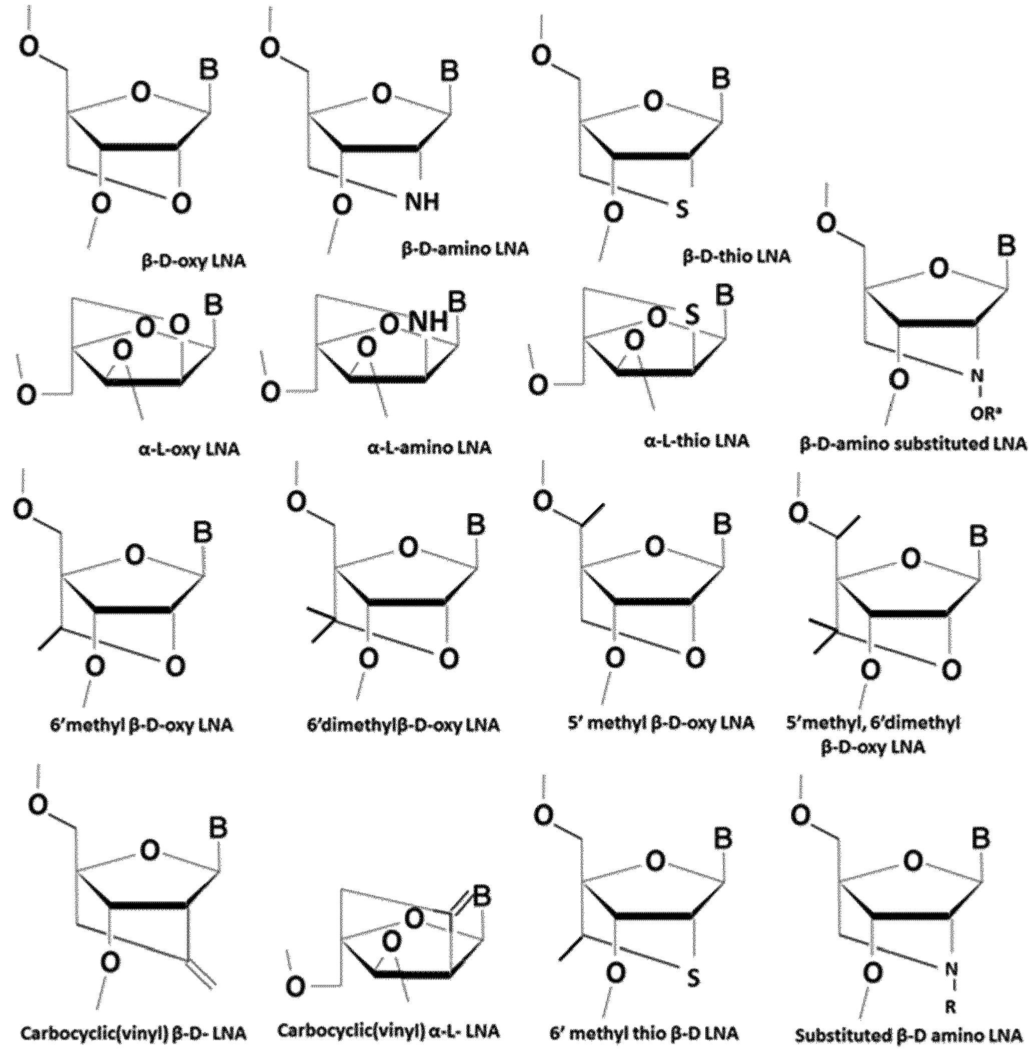

β-D-oxy LNA, β-D-amino LNA, β-D-thio LNA, α-L-oxy LNA, α-L-amino LNA, α-L-thio LNA, β-D-amino substituted LNA, 6'methyl β-D-oxy LNA, 6'dimethylβ-D-oxy LNA, 5' methyl β-D-oxy LNA, 5'methyl, 6'dimethyl β-D-oxy LNA, Carbocyclic(vinyl) β-D- LNA, Carbocyclic(vinyl) α-L- LNA, 6' methyl thio β-D LNA, Substituted β-D amino LNA

Particular LNA nucleosides are beta-D-oxy-LNA, 6'-methyl-beta-D-oxy LNA such as (S)-6'-methyl-beta-D-oxy-LNA (ScET) and ENA.

A particularly advantageous LNA is beta-D-oxy-LNA or methyl-beta-D-oxy-LNA (ScET).

***Gapmer***

The antisense oligonucleotide, or contiguous nucleotide sequence thereof, may be or comprise a gapmer, also termed gapmer oligonucleotide. The antisense gapmers are commonly used to inhibit a target nucleic acid via RNase H mediated degradation. A gapmer oligonucleotide comprises at least three distinct structural regions a 5'-flank, a gap and a 3'-flank, F-G-F' in the '5 -> 3' orientation. The "gap" region (G) comprises a stretch of contiguous DNA nucleotides which enable the oligonucleotide to recruit RNase H. The gap region is flanked by a 5' flanking region (F) comprising one or more sugar modified nucleosides, advantageously high affinity sugar modified nucleosides, and by a 3' flanking region (F') comprising one or more sugar modified nucleosides, advantageously high affinity sugar modified nucleosides. The one or more sugar modified nucleosides in region F and F' enhance the affinity of the oligonucleotide for the target nucleic acid (i.e. are affinity enhancing sugar modified nucleosides). In some embodiments, the one or more sugar modified nucleosides in region F and F' are 2' sugar modified nucleosides, such as high affinity 2' sugar modifications, such as independently selected from LNA and 2'-MOE.

In a gapmer design, the 5' and 3' most nucleosides of the gap region are DNA nucleosides, and are positioned adjacent to a sugar modified nucleoside of the 5' (F) or 3' (F') region respectively. The flanks may further be defined by having at least one sugar modified nucleoside at the end most distant from the gap region, i.e. at the 5' end of the 5' flank and at the 3' end of the 3' flank. Regions F-G-F' form a contiguous nucleotide sequence. Antisense oligonucleotides

or the contiguous nucleotide sequence thereof, may comprise a gapmer region of formula F-G-F'.

The overall length of the gapmer design F-G-F' may be, for example 12 to 32 nucleosides, such as 13 to 24, such as 14 to 22 nucleosides, Such as from 14 to17, such as 16 to18 nucleosides.

By way of example, the gapmer oligonucleotide can be represented by the following formulae:

$$F_{1-8}\text{-}G_{5-16}\text{-}F'_{1-8},$$

such as

$$F_{1-8}\text{-}G_{7-16}\text{-}F'_{2-8}$$

with the proviso that the overall length of the gapmer regions F-G-F' is at least 12, such as at least 14 nucleotides in length.

In an aspect of the invention the antisense oligonucleotide or contiguous nucleotide sequence thereof consists of or comprises a gapmer of formula 5'-F-G-F'-3', where region F and F' independently comprise or consist of 1- 8 nucleosides, of which 1-4 are 2' sugar modified and defines the 5' and 3' end of the F and F' region, and G is a region between 6 and 16 nucleosides which are capable of recruiting RNaseH.

Region G (gap region) of the gapmer is a region of nucleosides which enables the oligonucleotide to recruit RNaseH, such as human RNase H1, typically DNA nucleosides. RNaseH is a cellular enzyme which recognizes the duplex between DNA and RNA, and enzymatically cleaves the RNA molecule. Suitably gapmers may have a gap region (G) of at least 5 or 6 contiguous DNA nucleosides, such as 5 - 16 contiguous DNA nucleosides, such as 6 - 15 contiguous DNA nucleosides, such as 7-14 contiguous DNA nucleosides, such as 8 - 12 contiguous DNA nucleotides, such as 8 - 12 contiguous DNA nucleotides in length.

Alternatively, there are numerous reports of the insertion of a modified nucleoside which confers a 3' endo conformation into the gap region of gapmers, whilst retaining some RNaseH activity. Such gapmers with a gap region comprising one or more 3'endo modified nucleosides are referred to as "gap-breaker" or "gap-disrupted" gapmers, see for example WO2013/022984.

***LNA Gapmer***

An LNA gapmer is a gapmer wherein either one or both of region F and F' comprises or consists of LNA nucleosides. A beta-D-oxy gapmer is a gapmer wherein either one or both of region F and F' comprises or consists of beta-D-oxy LNA nucleosides.

In some embodiments the LNA gapmer is of formula: $[LNA]_{1-5}$-[region G] -$[LNA]_{1-5}$, wherein region G is as defined in the Gapmer region G definition.

***MOE Gapmers***

A MOE gapmers is a gapmer wherein regions F and F' consist of MOE nucleosides. In some embodiments the MOE gapmer is of design $[MOE]_{1-8}$-[Region G] $_{5-16}$-[MOE] $_{1-8}$, such as $[MOE]_{2-7}$-$[Region\ G]_{6-14}$-[MOE] $_{2-7}$, such as $[MOE]_{3-6}$-$[Region\ G]_{8-12}$-[MOE] $_{3-6}$, wherein region G is as defined in the Gapmer definition. MOE gapmers with a 5-10-5 design (MOE-DNA-MOE) have been widely used in the art.

***Mixed Wing Gapmer***

A mixed wing gapmer is an LNA gapmer wherein one or both of region F and F' comprise a 2' substituted nucleoside, such as a 2' substituted nucleoside independently selected from the group consisting of 2'-O-alkyl-RNA units, 2'-O-methyl-RNA, 2'-amino-DNA units, 2'-fluoro-DNA units, 2'-alkoxy-RNA, MOE units, arabino nucleic acid (ANA) units and 2'-fluoro-ANA units, such as a MOE nucleosides. In some embodiments wherein at least one of region F and F', or both region F and F' comprise at least one LNA nucleoside, the remaining nucleosides of region F and F' are independently selected from the group consisting of MOE and LNA. In some embodiments wherein at least one of region F and F', or both region F and F' comprise at least two LNA nucleosides, the remaining nucleosides of region F and F' are independently selected from the group consisting of MOE and LNA. In some mixed wing embodiments, one or both of region F and F' may further comprise one or more DNA nucleosides.

Mixed wing gapmer designs are disclosed in WO2008/049085 and WO2012/109395.

***Alternating Flank Gapmers***

Flanking regions may comprise both LNA and DNA nucleoside and are referred to as "alternating flanks" as

they comprise an alternating motif of LNA-DNA-LNA nucleosides. Gapmers comprising such alternating flanks are referred to as "alternating flank gapmers". "Alternative flank gapmers" are thus LNA gapmer oligonucleotides where at least one of the flanks (F or F') comprises DNA in addition to the LNA nucleoside(s). In some embodiments at least one of region F or F', or both region F and F', comprise both LNA nucleosides and DNA nucleosides. In such embodiments, the flanking region F or F', or both F and F' comprise at least three nucleosides, wherein the 5' and 3' most nucleosides of the F and/or F' region are LNA nucleosides.

***Region D' or D" in an oligonucleotide***

The oligonucleotide may in some embodiments comprise or consist of the contiguous nucleotide sequence of the oligonucleotide which is complementary to the target nucleic acid, such as the gapmer F-G-F', and further 5' and/or 3' nucleosides. The further 5' and/or 3' nucleosides may or may not be fully complementary to the target nucleic acid. Such further 5' and/or 3' nucleosides may be referred to as region D' and D" herein.

The addition of region D' or D" may be used for the purpose of joining the contiguous nucleotide sequence, such as the gapmer, to a conjugate moiety or another functional group. When used for joining the contiguous nucleotide sequence with a conjugate moiety is can serve as a biocleavable linker. Alternatively it may be used to provide exonucleoase protection or for ease of synthesis or manufacture.

Region D' and D" can be attached to the 5' end of region F or the 3' end of region F', respectively to generate designs of the following formulas D'-F-G-F', F-G-F'-D" or D'-F-G-F'-D". In this instance the F-G-F' is the gapmer portion of the oligonucleotide and region D' or D" constitute a separate part of the oligonucleotide.

Region D' or D" may independently comprise or consist of 1, 2, 3, 4 or 5 additional nucleotides, which may be complementary or non-complementary to the target nucleic acid. The nucleotide adjacent to the F or F' region is not a sugar-modified nucleotide, such as a DNA or RNA or base modified versions of these. The D' or D' region may serve as a nuclease susceptible biocleavable linker (see definition of linkers). In some embodiments the additional 5' and/or 3' end nucleotides are linked with phosphodiester linkages, and are DNA or RNA. Nucleotide based biocleavable linkers suitable for use as region D' or D" are disclosed in WO2014/076195, which include by way of example a phosphodiester linked DNA dinucleotide. The use of biocleavable linkers in polyoligonucleotide constructs is disclosed in WO2015/113922, where they are used to link multiple antisense constructs (e.g. gapmer regions) within a single oligonucleotide.

In one embodiment the oligonucleotide comprises a region D' and/or D" in addition to the contiguous nucleotide sequence which constitutes the gapmer.

In some embodiments, the oligonucleotide can be represented by the following formulae:

F-G-F'; in particular, $F_{1-8}$-$G_{5-16}$-$F'_{2-8}$
D'-F-G-F', in particular $D'_{1-3}$-$F_{1-8}$-$G_{5-16}$-$F'_{2-8}$
F-G-F'-D", in particular $F_{1-8}$-$G_{5-16}$-$F'_{2-8}$-$D"_{1-3}$
D'-F-G-F'-D", in particular $D'_{1-3}$- $F_{1-8}$-$G_{5-16}$-$F'_{2-8}$-$D"_{1-3}$

In some embodiments the internucleoside linkage positioned between region D' and region F is a phosphodiester linkage. In some embodiments the internucleoside linkage positioned between region F' and region D" is a phosphodiester linkage.

**Total mers**

In some embodiments, the oligomer or contiguous nucleotide sequence thereof consists of a contiguous sequence of nucleoside analogues, such as affinity enhancing nucleoside analogues - referred to herein is as a 'totalmer'.

A totalmer is a single stranded oligomer, or contiguous nucleotide sequence thereof, which does not comprise DNA or RNA nucleosides, and as such only comprises nucleoside analogue nucleosides. The oligomer, or contiguous nucleotide sequence thereof, maybe a totalmer - indeed various totalmer designs are highly effective as therapeutic oligomers, particularly when targeting microRNA (antimiRs) or as splice switching oligomers (SSOs).

In some embodiments, the totalmer comprises or consists of at least one XYX or YXY sequence motif, such as a repeated sequence XYX or YXY, wherein X is LNA and Y is an alternative (i.e. non LNA) nucleotide analogue, such as a 2'-OMe RNA unit and 2'-fluoro DNA unit. The above sequence motif may, in some embodiments, be XXY, XYX, YXY or YYX for example.

In some embodiments, the totalmer may comprise or consist of a contiguous nucleotide sequence of between 8 and 16 nucleotides, such as 9, 10, 11, 12, 13, 14, or 15 nucleotides, such as between 8 and 12 nucleotides.

In some embodiments, the contiguous nucleotide sequence of the totalmer comprises of at least 30%, such as at least 40%, such as at least 50%, such as at least 60%, such as at least 70%, such as at least 80%, such as at

least 90%, such as 95%, such as 100% LNA units. The remaining units may be selected from the non-LNA nucleotide analogues referred to herein in, such as those selected from the group consisting of 2'-O_alkyl-RNA unit, 2'-OMe-RNA unit, 2'-amino-DNA unit, 2'-fluoro-DNA unit, LNA unit, PNA unit, HNA unit, INA unit, and a 2'MOE RNA unit, or the group of 2'-OMe RNA unit and 2'-fluoro DNA unit.

In some embodiments the totalmer consist or comprises of a contiguous nucleotide sequence which consists only of LNA units.

***Mixmers***

The term 'mixmer' refers to oligomers which comprise both DNA nucleosides and sugar modified nucleosides, wherein there are insufficient length of contiguous DNA nucleosides to recruit RNaseH. Suitable mixmers may comprise up to 3 or up to 4 contiguous DNA nucleosides. In some embodiments the mixmers, or contiguous nucleotide sequence thereof, comprise alternating regions of sugar modified nucleosides, and DNA nucleosides. By alternating regions of sugar modified nucleosides which form a RNA like (3'endo) conformation when incorporated into the oligonucleotide, with short regions of DNA nucleosides, non-RNaseH recruiting oligonucleotides may be made. Advantageously, the sugar modified nucleosides are affinity enhancing sugar modified nucleosides.

Oligonucleotide mixmers are often used to provide occupation based modulation of target genes, such as splice modulators or microRNA inhibitors.

In some embodiments the sugar modified nucleosides in the mixmer, or contiguous nucleotide sequence thereof, comprise or are all LNA nucleosides, such as (S)cET or beta-D-oxy LNA nucleosides.

In some embodiments all of the sugar modified nucleosides of a mixmer comprise the same sugar modification, for example they may all be LNA nucleosides, or may all be 2'O-MOE nucleosides. In some embodiments the sugar modified nucleosides of a mixmer may be independently selected from LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments the oligonucleotide comprises both LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments, the oligonucleoitide comprises LNA nucleosides and 2'-O-MOE nucleosides. In some embodiments, the oligonucleotide comprises (S)cET LNA nucleosides and 2'-O-MOE nucleosides.

In some embodiments the mixmer, or continguous nucleotide sequence thereof, comprises only LNA and DNA nucleosides, such LNA mixmer oligonucleotides which may for example be between 8 - 24 nucleosides in length (see for example, WO2007112754, which discloses LNA antmiR inhibitors of microRNAs).

***Conjugate***

The term conjugate as used herein refers to an oligonucleotide which is covalently linked to a non-nucleotide moiety (conjugate moiety or region C or third region).

Conjugation of the oligonucleotide to one or more non-nucleotide moieties may improve the pharmacology of the oligonucleotide, e.g. by affecting the activity, cellular distribution, cellular uptake or stability of the oligonucleotide. In some embodiments the conjugate moiety modify or enhance the pharmacokinetic properties of the oligonucleotide by improving cellular distribution, bioavailability, metabolism, excretion, permeability, and/or cellular uptake of the oligonucleotide. In particular the conjugate may target the oligonucleotide to a specific organ, tissue or cell type and thereby enhance the effectiveness of the oligonucleotide in that organ, tissue or cell type. A the same time the conjugate may serve to reduce activity of the oligonucleotide in non-target cell types, tissues or organs, e.g. off target activity or activity in non-target cell types, tissues or organs.

In some embodiments the conjugate group is or comprises a polar moiety. Polar conjugates include carbohydrate conjugates, protein conjugates, peptide conjugates, polymer conjugates, such as PEG conjugates. In some embodiments the conjugate moiety is or comprises a N-acetylgalactosamine residue. In some embodiments the conjugate is or comprises a reporter group, such as a fluorphore.

Oligonucleotide conjugates and their synthesis has also been reported in comprehensive reviews by Manoharan in Antisense Drug Technology, Principles, Strategies, and Applications, S.T. Crooke, ed., Ch. 16, Marcel Dekker, Inc., 2001 and Manoharan, Antisense and Nucleic Acid Drug Development, 2002, 12, 103.

In an embodiment, the non-nucleotide moiety (conjugate moiety) is selected from the group consisting of carbohydrates (e.g. GalNAc), cell surface receptor ligands, drug substances, hormones, lipophilic substances, polymers, proteins, peptides, toxins (e.g. bacterial toxins), vitamins, viral proteins (e.g. capsids) or combinations thereof.

In some embodiments, the conjugate moieties are those capable of binding to the asialoglycoprotein receptor (ASGPR). In particular tri-valent N-acetylgalactosamine conjugate moieties are suitable for binding to the ASGPR (GalN-

Ac) see for example WO 2014/076196, WO 2014/207232 and WO 2014/179620. Such conjugates serve to enhance uptake of the oligonucleotide to the liver while reducing its presence in the kidney, thereby increasing the liver/kidney ratio of a conjugated oligonucleotide compared to the unconjugated version of the same oligonucleotide.

### *Linkers*

A linkage or linker is a connection between two atoms that links one chemical group or segment of interest to another chemical group or segment of interest via one or more covalent bonds. Conjugate moieties can be attached to the oligonucleotide directly or through a linking moiety (e.g. linker or tether). Linkers serve to covalently connect a third region, e.g. a conjugate moiety (Region C), to a first region, e.g. an oligonucleotide or contiguous nucleotide sequence complementary to the target nucleic acid (region A).

In some embodiments of the invention the conjugate or oligonucleotide conjugate may optionally, comprise a linker region (second region or region B and/or region Y) which is positioned between the oligonucleotide or contiguous nucleotide sequence complementary to the target nucleic acid (region A or first region) and the conjugate moiety (region C or third region). Region B refers to biocleavable linkers comprising or consisting of a physiologically labile bond that is cleavable under conditions normally encountered or analogous to those encountered within a mammalian body. Conditions under which physiologically labile linkers undergo chemical transformation (e.g., cleavage) include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic enzymes or hydrolytic enzymes or nucleases. In one embodiment the biocleavable linker is susceptible to S1 nuclease cleavage. In a preferred embodiment the nuclease susceptible linker comprises between 1 and 10 nucleosides, such as 1, 2, 3, 4, 5, 6, 7, 8, 9 or 10 nucleosides, more preferably between 2 and 6 nucleosides and most preferably between 2 and 4 linked nucleosides comprising at least two consecutive phosphodiester linkages, such as at least 3 or 4 or 5 consecutive phosphodiester linkages. Preferably the nucleosides are DNA or RNA. Phosphodiester containing biocleavable linkers are described in more detail in WO 2014/076195. Region Y refers to linkers that are not necessarily biocleavable but primarily serve to covalently connect a conjugate moiety (region C or third region), to an oligonucleotide (region A or first region). The region Y linkers may comprise a chain structure or an oligomer of repeating units such as ethylene glycol, amino acid units or amino alkyl groups The oligonucleotide conjugates can be constructed of the following regional elements A-C, A-B-C, A-B-Y-C, A-Y-B-C or A-Y-C. In some embodiments the linker (region Y) is an amino alkyl, such as a C2 - C36 amino alkyl group, including, for example C6 to C12 amino alkyl groups. In a preferred embodiment the linker (region Y) is a C6 amino alkyl group.

## DETAILED DESCRIPTION OF THE INVENTION

### The Oligonucleotide

In some embodiments the oligonucleotide has a length of 10 - 50 nucleotides, such as 14 - 25 nucleotides. In some embodiments the oligonucleotide has a length of 14, 15, 16, 17, 18, 19 or 20 nucleotides.

As illustrated in the examples the methods of the present invention are particularly useful in formulating phosphorothioate oligonucleotides, *i.e.* oligonucleotides which comprise phosphorothioate internucleotide linkages. Indeed, the examples illustrate that compounds which comprise phosphorothioate internucleosides linkages have a linear correlation based on DVS results, even surpassing phosphodiester linked oligonucleotides. This is surprising as the sulfur atom in the phosphorothioate linkages is considerably weaker in its ability to form hydrogen bonds to water (Platts et al., J. Am. Chem. Soc., 1996, 118 (11), pp 2726-2733). Other sulfur containing internucleoside likages which may be present in the oligonucleotide include phosphorodithioate and phosphorotrithioate internucleoside linkages.

The examples further illustrate that the methods of the invention are particularly applicable to oligonucleotides which comprise one or more sugar modified nucleosides, such as one or more sugar modified nucleosides which comprise an electronegative 2' group (e.g. substituent or in the case of LNA a 4' - 2' biradical). Such sugar modified nucleosides are often used in antisense oligonucleotides, PCR primers and hybridization probes, where they enhance the binding affinity to complementary nucleotide sequences.

In some embodiments, the oligonucleotide is or comprises an antisense oligonucleotide, wherein the antisense oligonucleotide comprises one or more sugar modified nucleosides, such as one or more sugar modified nucleoside independently selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, 2'-F-ANA nucleoside, and LNA nucleoside(s).

In some embodiments, the oligonucleotide is or comprises an antisense oligonucleotide, wherein the antisense oligonucleotide comprises one or more sugar modified nucleosides, such as one or more sugar modified nucleoside independently selected from the group consisting of 2'-O-methoxyethyl and LNA.

In some embodiments, the oligonucleotide comprises one or more sugar modified nucleoside, such as one or more LNA nucleosides and/or one or more 2'-methoxyethyl (2-MOE) nucleosides, and phosphorothioate internucleoside linkages.

In some embodiments, the oligonucleotide comprises one or more sugar modified nucleoside, such as one or more LNA nucleosides and/or one or more 2'-methoxyethyl (2-MOE) nucleosides, and further comprises both phosphorothioate and phosphodiester internucleoside linkages.

In some embodiments the oligonucleotide is or comprises a gapmer oligonucleotide, such as a LNA gapmer (where region F and F' both comprise LNA nucleosides), a MOE gapmers (where region F and F' both comprise MOE nucleosides). A mixed wing gapmer, an alternating flank gapmer, and a gap-breaker oligonucleotide. In some embodiments the internucleoside linkages within the nucleotides of the gapmer are all phosphorothioate. As illustrated in the examples, the phosphorothioate oligonucleotide region (e.g. gapmer region) may be flanked by one or more phosphodiester linked nucleosides (e.g. described as region D herein). Gapmers or gapmer regions are typically at least 12 nucleotides in length, and may be as long as 24 or 26 nucleotides, however are more typically 15 - 20 nucleotides in length. In some embodiments, the oligonucleotide is a totalmer or mixmer oligonucleotide.

In some embodiments the oligonucleotide comprises a fluorescent label (also referred to as a fluorescent dye). In some embodiments the oligonucleotide comprises a quencher.

In some embodiments the oligonucleotide comprises a carbohydrate conjugate group, such as a GalNAc moiety.

***The Atmosphere***

The method of the invention comprises a period where the oligonucleotide compound salt is exposed to an atmosphere for a time period to allow for the water content (hydration level) of the oligonucleotide compound salt to sufficiently equilibrate with the atmosphere (the time period may be referred to as the acclimatization period.

In some embodiments, the atmosphere used for the acclimatization period is an atmosphere which has a predetermined RH level of between about 10% - about 70%, or predetermined RH level of between about 10 - 60%.

In some embodiments, the RH of the local atmosphere is measured during the acclimatization period in order to determine the RH of the atmosphere. In some embodiments the atmosphere during the acclimatization period is the atmosphere in the room (local atmosphere).

RH levels of the atmosphere of 10 - 50% are advantageous as above 50% the absorption or sorption rates are slower, requiring a longer acclimatization period (e.g. at least about 4 hours).

***Time period for absorption and sorption (Acclimatization Period)***

As illustrated in the examples, the rate of water gain /loss of an oligonucleotide is dependent upon whether the relative humidity of the atmosphere (RH) is higher or lower than that in which the oligonucleotide compound salt has been stored, and the difference between the RH. Transfer of the oligonucleotide compound salt from into an atmosphere with a higher RH will result in absoption, whereas transfer of the oligonucleotide compound salt into an atmosphere with a lower RH will result in sorption. The rate of sorption is typically slower than the rate of absorption.

Typically, a period of at least about 2 - 4 hours is sufficient to allow for the absorption/sorbtion to be sufficiently complete to allow for the determination of the dry weight of the oligonucleotide to be determined by the present method.

In some embodiments, in step b, the solid powdered form of the oligonucleotide compound salt is exposed to an atmosphere for a period of at least about 2 hours, wherein the atmosphere has a relative humidity (RH) of between about 10 - about 70%, such as a known relative humidity (RH) of between about 10% and about 60%, such as a known relative humidity (RH) of between about 20% and about 50%.

In some embodiments, in step b, the solid powdered form of the oligonucleotide compound salt is exposed to an atmosphere for a period of at least about 3 hours, wherein the atmosphere has a known relative humidity (RH) of between about 10 - about 70%, such as a known relative humidity (RH) of between about 10% and about 60%, such as a known relative humidity (RH) of between about 20% and about 50%.

In some embodiments, in step b, the solid powdered form of the oligonucleotide compound salt is exposed to an atmosphere for a period of at least about 4 hours, wherein the atmosphere has a known relative humidity (RH) of between about 10 - about 70%, such as a known relative humidity (RH) of between about 10% and about 60%, such as a known relative humidity (RH) of between about 20% and about 50%.

In some embodiments, in step b, the solid powdered form of the oligonucleotide compound salt is exposed to an atmosphere for a period of at least about 5 hours, wherein the atmosphere has a known relative humidity (RH) of between about 10 - about 70%, such as a known relative humidity (RH) of between about 10% and about 60%, such as a known relative humidity (RH) of between about 20% and about 50%.

In some embodiments, in step b, the solid powdered form of the oligonucleotide compound salt is exposed to an atmosphere for a period of at least about 6 hours, wherein the atmosphere has a known relative humidity (RH) of

between about 10 - about 70%, such as a known relative humidity (RH) of between about 10% and about 60%, such as a known relative humidity (RH) of between about 20% and about 50%.

In some embodiments, in step b, the solid powdered form of the oligonucleotide compound salt is exposed to an atmosphere for a period of at least about 8 hours (such as overnight), wherein the atmosphere has a known relative humidity (RH) of between about 10 - about 70%, such as a known relative humidity (RH) of between about 10% and about 60%, such as a known relative humidity (RH) of between about 20% and about 50%.

In some embodiments, in step b, the solid powdered form of the oligonucleotide compound salt is exposed to an atmosphere for a period of at least about 1 hour, wherein the atmosphere has a known relative humidity (RH) of between about 10 - about 70%, such as a known relative humidity (RH) of between about 10% and about 60%, such as a known relative humidity (RH) of between about 20% and about 50%.

***Conjugate Groups***

As illustrated in the examples, the methods of the invention may be used in determining the dry weight or number of moles of oligonucleotide conjugates. The examples illustrate that the incorporation of hydrophilic conjugates, which comprise multiple sides for hydrogen bond formation (hydration sites), does not disrupt the linear correlation based on DVS results as compared to an equivalent unconjugated compound. Carbohydrate conjugates, such as GalNAc conjugates retain excellent linearity between 10 - 70% RH, equivalent to the unconjugated oligonucleotide equivalent. In comparison, the use of a polyphenolic conjugate (exemplified using 6-FAM (fluorescein), provided also a excellent linearity between 10 - 60% RH, equivalent to the unconjugated oligonucleotide equivalent.

In some embodiments, the conjugate group is a hydrophilic conjugate group which comprises two or more oxygen atoms which are available for hydrogen bonding (hydration).

***Carbohydrate conjugate moieties***

As is illustrated in the examples, the present invention provides methods for determining the dry weight or number of moles of an oligonucleotide including oligonucleotide conjugates. The examples illustrate that the methods of the invention are particularly useful in determining the dry weight/number of moles of oligonucleotide conjugates which comprise a hydrophilic conjugate moiety, such as a carbohydrate conjugate moiety (non-nucleosidic). In some embodiments the conjugate moiety comprises one or more C6 sugar residues (hexoses).

In some embodiments the carbohydrate conjugate moiety comprises a carbohydrate group selected from the group consisting of galactose, lactose, n-acetylgalactosamine (galNAc), mannose and mannose-6-phosphate group.

Carbohydrate conjugates may be used to enhance delivery or activity in a range of tissues, such as liver and/or muscle. See, for example, EP1495769, WO99/65925, Yang et al., Bioconjug Chem (2009) 20(2): 213-21. Zatsepin & Oretskaya Chem Biodivers. (2004) 1(10): 1401-17.

In some embodiments the carbohydrate conjugate moiety is multivalent, such as, for example 2, 3 or 4 identical or non-identical carbohydrate moieties may be covalently joined to the oligonucleotide, optionally via a linker or linkers.

In some embodiments, the conjugate moiety is or may comprise mannose or mannose-6-phosphate. This is particular useful for targeting muscle cells, see for example US 2012/122801. Conjugate moieties capable of binding to the asialoglycoprotein receptor (ASGPR) are particular useful for targeting hepatocytes in liver. A conjugate may comprise an oligonucleotide and an asialoglycoprotein receptor targeting conjugate moiety. The asialoglycoprotein receptor (ASGPR) conjugate moiety comprises one or more carbohydrate moieties capable of binding to the asialoglycoprotein receptor (ASPGR targeting moieties) with affinity equal to or greater than that of galactose. The affinities of numerous galactose derivatives for the asialoglycoprotein receptor have been studied (see for example: Jobst, S.T. and Drickamer, K. JB.C. 1996, 271, 6686) or are readily determined using methods typical in the art.

In one embodiment the conjugate moiety comprises at least one asialoglycoprotein receptor targeting moiety selected from group consisting of galactose, galactosamine, N-formyl-galactosamine, N-acetylgalactosamine, N-propionyl-galactosamine, N-n-butanoyl-galactosamine and N-isobutanoylgalactosamine. Advantageously the asialoglycoprotein receptor targeting moiety is N-acetylgalactosamine (GalNAc).

To generate the ASGPR conjugate moiety the ASPGR targeting moieties (preferably GalNAc) can be attached to a conjugate scaffold. Generally the ASPGR targeting moieties can be at the same end of the scaffold. In one embodiment the conjugate moiety consists of two to four such as three terminal GalNAc moieties linked to a spacer which links each GalNAc moiety to a brancher molecule that can be conjugated to the antisense oligonucleotide.

In a further embodiment the conjugate moiety is mono-valent, di-valent, tri-valent or tetra-valent with respect to asialoglycoprotein receptor targeting moieties. Advantageously the asialoglycoprotein receptor targeting moiety comprises N-acetylgalactosamine (GalNAc) moieties. The ASPGR targeting scaffold which constitute the conjugate moiety can for example be generated by linking the GalNAc moiety to the spacer through its C-I carbon. A preferred spacer is a flexible hydrophilic spacer (U.S. Patent 5885968; Biessen et al. J. Med. Chern. 1995 Vol. 39 p. 1538-1546). A preferred

flexible hydrophilic spacer is a PEG spacer. A preferred PEG spacer is a PEG3 spacer. The branch point can be any small molecule which permits attachment of two to three GalNAc moieties or other asialoglycoprotein receptor targeting moieties and further permits attachment of the branch point to the oligonucleotide, such constructs are termed GalNAc clusters or GalNAc conjugate moieties. An exemplary branch point group is a di-lysine. A di-lysine molecule contains three amine groups through which three GalNAc moieties or other asialoglycoprotein receptor targeting moieties may be attached and a carboxyl reactive group through which the di-lysine may be attached to the oligomer. Khorev, et al 2008 Bioorg. Med. Chem. Vol 16, pp. 5216 also describes the synthesis of a suitable trivalent brancher. Other commercially available branchers are 1,3-bis-[5-(4,4'-dimethoxytrityloxy)pentylamido]propyl-2-[(2-cyanoethyl)-(N,N-diisopropyl)] phosphoramidite (Glen Research Catalogue Number: 10-1920-xx); tris-2,2,2-[3-(4,4'-dimethoxytrityloxy)propyloxymethyl]ethyl-[(2-cyanoethyl)-(N,N-diisopropyl)]-phosphoramidite (Glen Research Catalogue Number: 10-1922-xx); and tris-2,2,2-[3-(4,4'-dimethoxytrityloxy)propyloxymethyl]methyleneoxypropyl-[(2-cyanoethyl)-(N,N-diisopropyl)]-phosphoramidite; and 1-[5-(4,4'-dimethoxy-trityloxy)pentylamido]-3-[5-fluorenomethoxy-carbonyl-oxy-pentylamido]-propyl-2-[(2-cyanoethyl)-(N,N-diisopropyl)]-phosphoramidite (Glen Research Catalogue Number: 10-1925-xx); as well as small peptides with GalNAc moieties attached such as Tyr-Glu-Glu-(aminohexyl GalNAc)3 (YEE(ahGalNAc)3; a glycotripeptide that binds to asialoglycoprotein receptor on hepatocytes, see, e.g., Duff, et al., Methods Enzymol, 2000, 313, 297); lysine-based galactose clusters (e.g., L3G4; Biessen, et al., Cardovasc. Med., 1999, 214); and cholane-based galactose clusters (e.g., carbohydrate recognition motif for asialoglycoprotein receptor).

The ASGPR conjugate moiety, in particular a trivalent GalNAc conjugate moiety, may be attached to the 3'- or 5'-end of the oligonucleotide using methods known in the art. In one embodiment the ASGPR conjugate moiety is linked to the 5'-end of the oligonucleotide.

One or more linkers may be inserted between the conjugate moiety (such as at the brancher molecule) and the oligonucleotide. It is advantageous to have a biocleavable linker between the conjugate moiety and the antisense oligonucleotide, optionally in combination with a non-cleavable linker such as a C6 linker. The linker(s) may be selected from the linkers described in the "Definitions" section under "Linkers" in particular biocleavable region D' or D" linkers are advantageous.

In some embodiments, the conjugate group comprises one or more N-acetylgalactosamine residues (GalNAc):

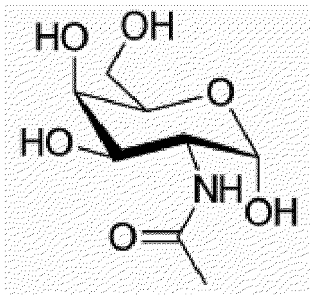

In some embodiments, the conjugate moiety comprises multiple N-acetylgalactosamine residues (GalNAc complex). In some embodiments the N-acetylgalactosamine residue(s) are covalently attached to a polymer group, such as a PEG linker (PEG comprises further hydrogen bonding sites). Multiple GalNAcs (or e.g. GalNAc-PEG moieties) may be covently joined together via linking moieties, such as via a peptide linker, such as a poly-lysine linkers.

In some embodiments the galNAc conjugate is covalently attached to the 5' terminus of the oligonucleotide, optionally via a linker group such as a C6 linker.

In one embodiment the conjugate moiety is or comprises a tri-valent N-acetylgalactosamine (GalNAc), such as a GalNAc moiety selected from those shown below (the wavy line represents a covalent bond to the 5' or 3' terminus of the oligonucleotide or to a linker positioned between the conjugate moiety and the 5' or 3' terminus of the oligonucleotide):

Conj 1=

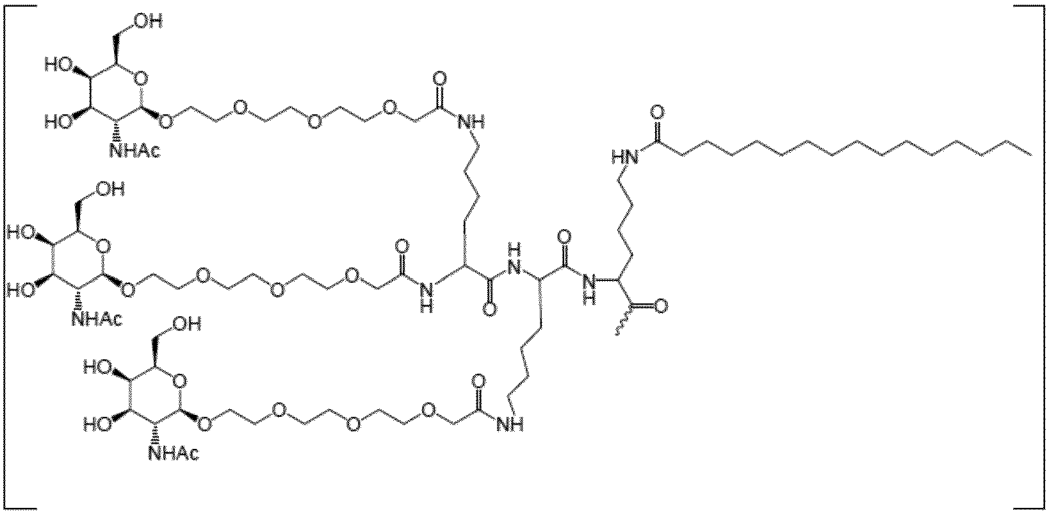

Conj 2=

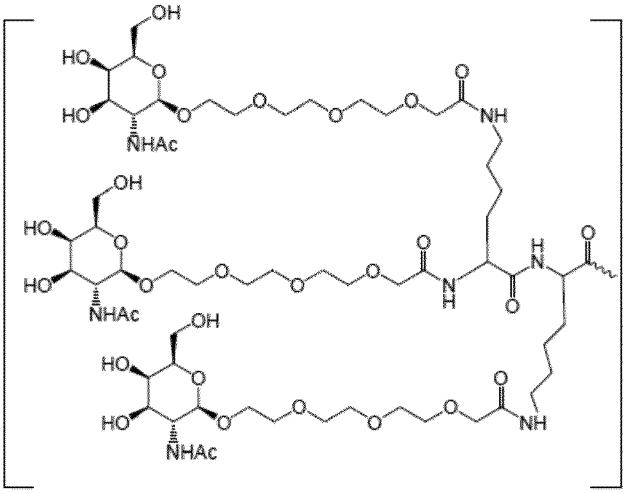

Conj 3=

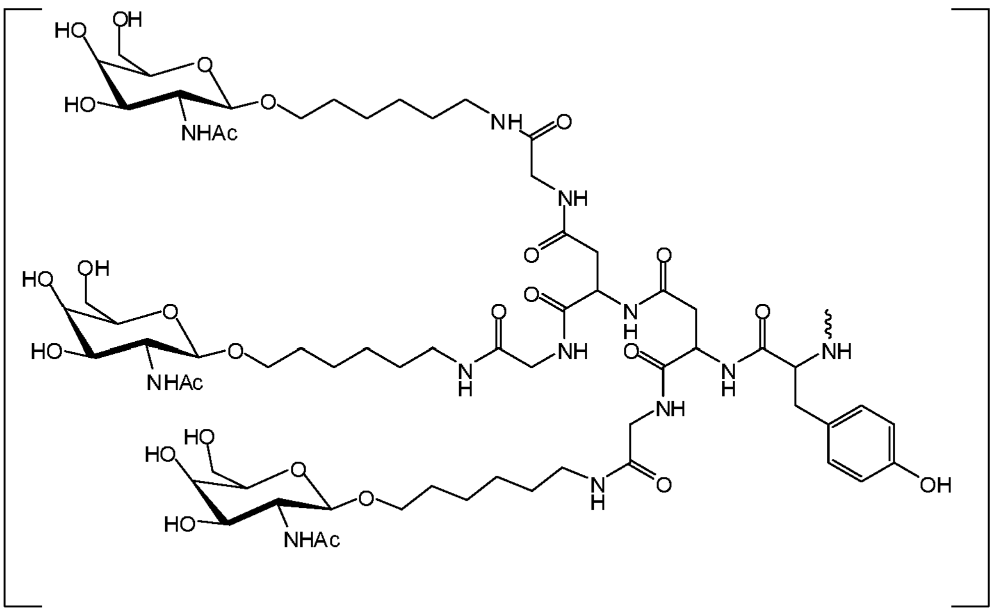

Conj 4=

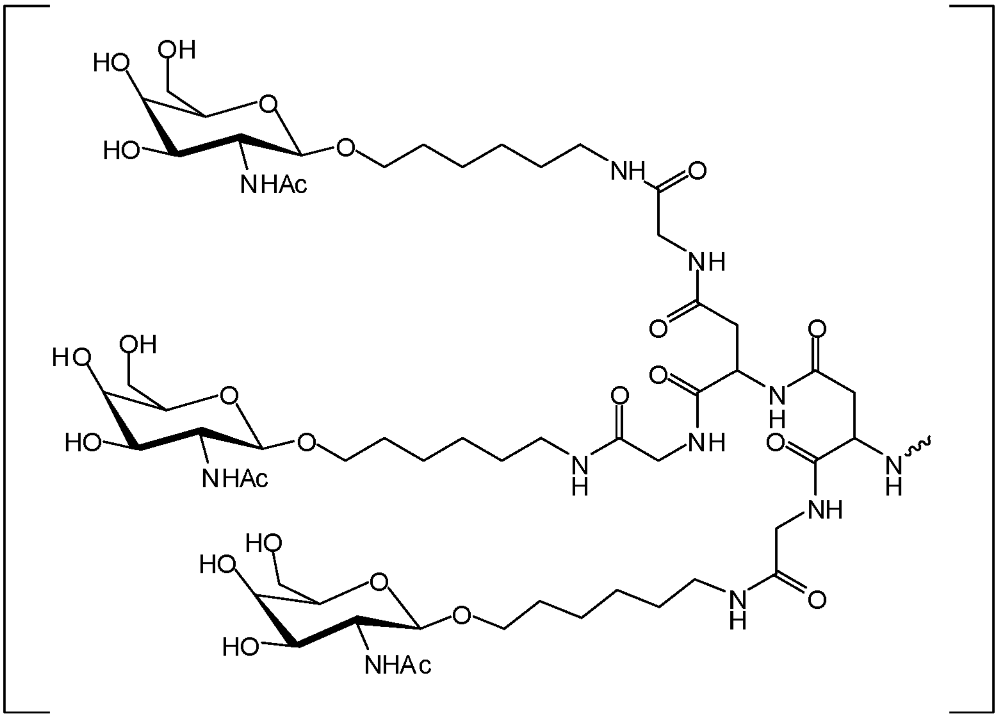

Conj 1a=

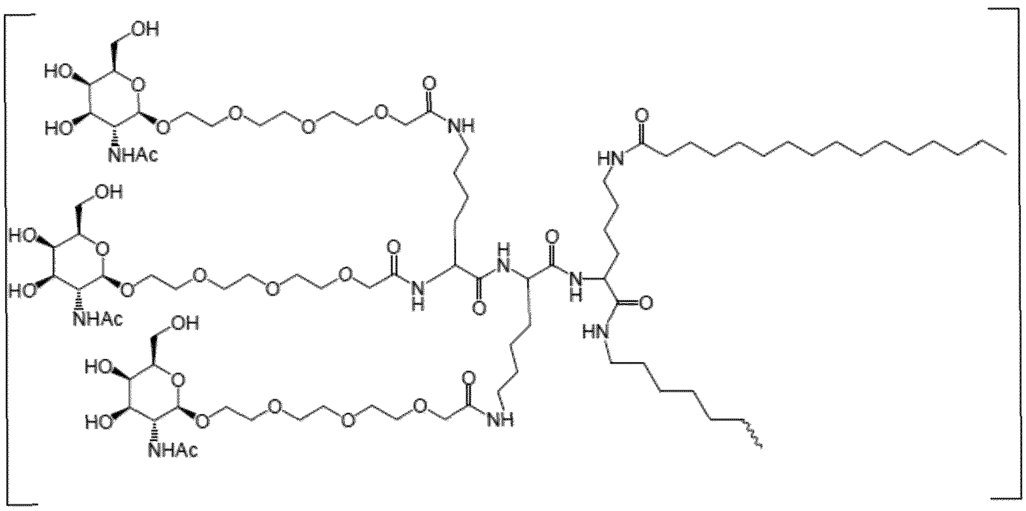

Conj 2a=

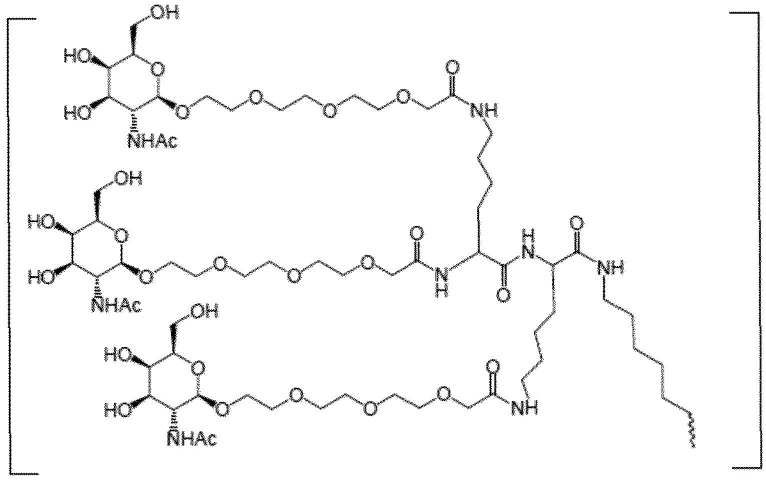

Conj 3a=

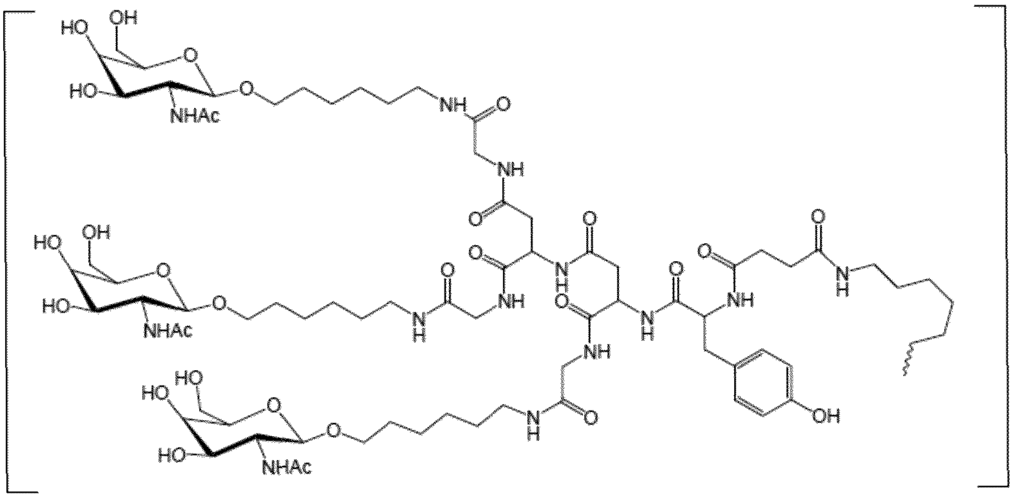

Conj 4a=

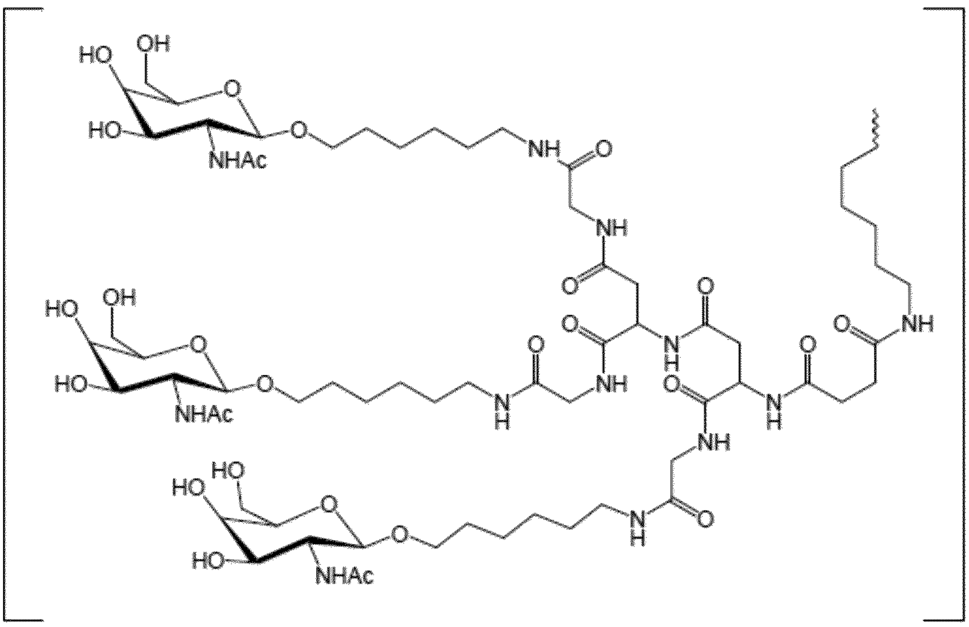

**Conj5**

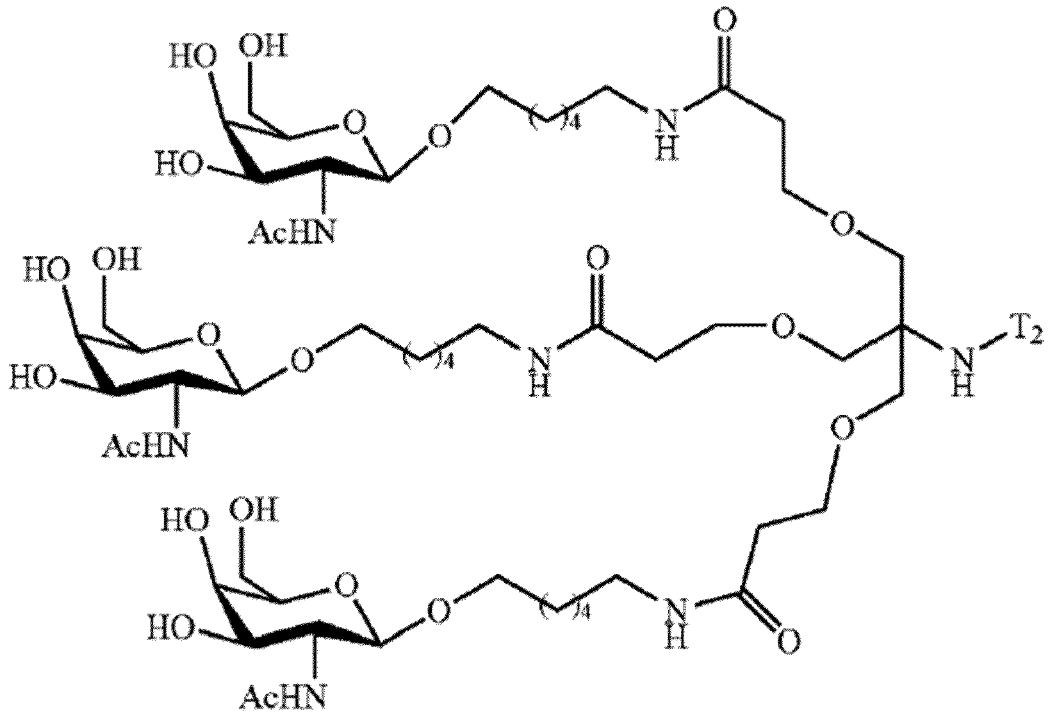

Wherein T2 is the oligonucleotide or a linker linking the GalNAc moiety to the oligonucleotide, as described in WO2014/179620 which discloses the use of a 3' conjugated GalNAc moiety on antisense oligonucleotides.

WO2016/055601 described the introduction of GalNAc conjugate moieties onto a oligonucleotide using the phosphoramidite approach, for example:

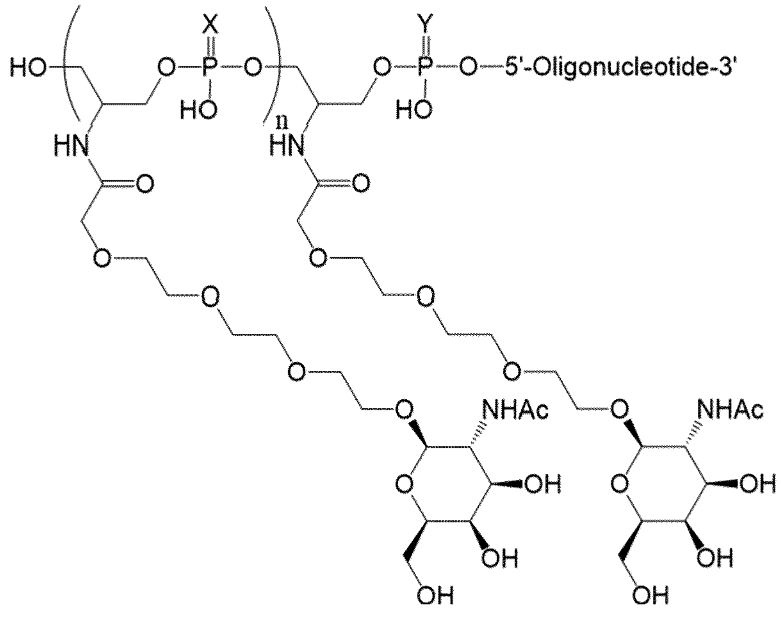

***Polymer conjugate moieties***

In some embodiments, the conjugate moiety is or comprises a polymer. Polymers include but is not limited to hydrophilic polymers such as polyalkylene oxide, polyethylglycol (PEG) and/or polypropylene glycol (PPG) - see WO 2008/034123 polyamines, polypeptides, polymethacrylates (e.g., hydroxylpropyl methacrylate (HPMA)), poly(L-lactide), poly(DL lactide-co-glycolide (PGLA), polyacrylic acids, polyethylenimines (PEI), polyalkylacrylic acids, polyurethanes,

polyacrylamides, N- alkylacrylamides, polyspermine (PSP), polyethers, cyclodextrins, derivatives thereof and co-polymers thereof.

In some embodiments, the conjugate moiety comprises PEG group or a PEG spacer.

## Reporter Groups

In some embodiments the conjugate moiety is or comprises a reporter group, such as a fluorphore. ememplary fluorophores include6-carbofluorescein, 6-carboxytetramethylrhodamine, Cy3, and coumarin:

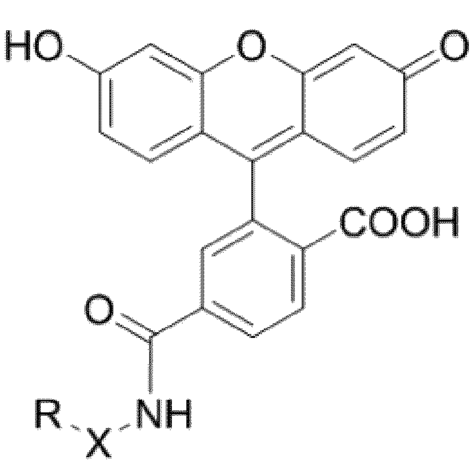

6-Carboyfluorescein

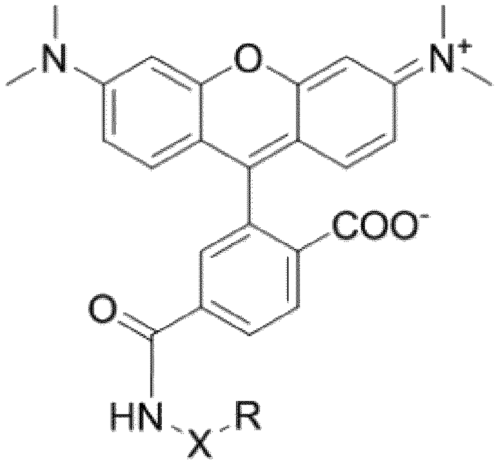

6-Carboxytetramethylrhodamine (TAMRA)

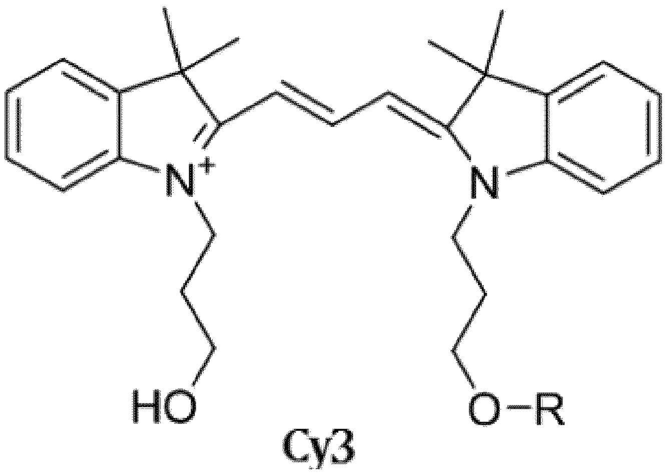

Cy3

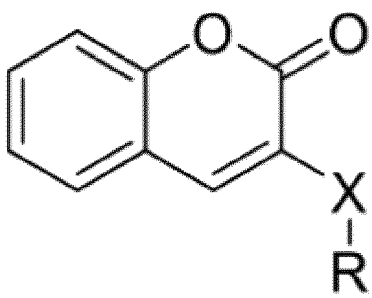

Coumarin

Wherein X is a linker and R is the oligonucleotide.

Fluorescent labelled oligonucleotides are routinely used in qPCR for example, often in combination with quencher molecules. In some embodiments, the oligonucleotide comprises a quencher moiety group, for example dabcyl, BHQ2, IB FQ and IQ4, for example.

Reporter groups are typically incorporated into oligonucleotides as a phosphoramidite or post oligonucleotide synthesis e.g. via an amino group/ester reaction.

## EXAMPLES

### Compound Table

The following compounds were used for DVS analysis. The compounds were selected based on their difference in length, sequence, presence of different conjugates, and presence of different nucleotide analogues. The compounds were also selected based on presence of phosphodiester and phosphorothioate linkages in different combination.

| Compound Number (SEQ ID) | Full Sequence | No. nucleotides | Description |
|---|---|---|---|
| 1 | 5’-GN2-C6$_o$c$_o$a$_o$GCGtaaagagaGG | 15 | LNA/DNA, PO/PS backbone |
| 2 | CTcccaacgtgcgCCa | 16 | LNA/DNA, PS backbone |
| 3 | 5’-GN2-C6$_o$c$_o$a$_o$GCGtaaagagaGGT | 16 | LNA/DNA, PO/PS backbone |
| 4 | 5’-GN2-C6$_o$c$_o$a$_o$GCGtaaagagaGG | 15 | LNA/DNA, PO/PS backbone |
| 5 | 5’-GN2-C6$_o$c$_o$a$_o$GCGtaaagagAGG | 15 | LNA/DNA, PO/PS backbone |
| 3 | 5’-GN2-C6nnNNnnnnnnnnnnnNNNN | 19 | LNA/DNA, PO/PS backbone |
| 7 | ACgtctcgtaGT | 12 | LNA/DNA, PS backbone |
| 8 | GCCTCagtctgcttcGCACC | 20 | LNA/DNA, PS backbone |
| 9 | 5’-GN2-C6GCtgtgtgagcttGG | 15 | LNA/DNA, PS backbone |
| 10 | GCGtaaagagaGGT | 14 | LNA/DNA, PS backbone |
| 11 | 5’-AM-C6$_o$c$_o$a$_o$CGAaccactgaaCAA | 17 | LNA/DNA, PO/PS backbone |
| 12 | 5’-G N2-C6$_o$c$_o$a$_o$GCGtaaagagaGGT | 16 | LNA/DNA, PO/PS backbone |
| 13 | ATGtgtctcttctcttcGCC | 20 | LNA/DNA, PS backbone |
| 14 | AgaCaccaataacaTtAGCA | 20 | LNA/DNA, PS backbone |
| 15 | GctgattagagagaggtCCC | 20 | MOE/DNA, PS backbone |
| 16 | AATgctacaaaacCCAC6SH | 16 | LNA/DNA, PS backbone |
| 17 | 5’-GN2-C6$_o$c$_o$a$_o$GAACaaatggcactaGTAA | 21 | LNA/DNA, PO/PS backbone |
| 18 | ttgaagatgtagatgt | 16 | DNA, PS backbone |
| 19 | A$_o$g$_o$a$_o$C$_o$a$_o$c$_o$c$_o$a$_o$a$_o$t$_o$a$_o$a$_o$c$_o$a$_o$T$_o$t$_o$A$_o$G$_o$C $_o$A | 20 | LNA/DNA, PO backbone |

(continued)

| Compound Number (SEQ ID) | Full Sequence | No. nucleotides | Description |
|---|---|---|---|
| 20 | 5'-FAMAGTtataatccaGCT | 15 | LNA/DNA, PS backbone |

Phosphodiester internucleoside linkages are represented by a subscript $_o$, otherwise all internucleoside linkages are phosphorothioate internucleoside linkages; non italic capital letters represent beta-D-oxy LNA nucleoside, all beta-D-oxy LNA C are 5-methyl cytosine beta-D-oxy LNA; italic capital letters represent 2'-O-methoxyethyl RNA nucleoside, lower case letters represent DNA nucleosides; 5'-GN2-C6 represents a trivalent GalNAc conjugate with a C6 alkyl linker covalently attached to the 5' position of the 5' terminal nucleoside of the oligonucleotide sequence; 6SH represents a 3' terminal thiol; 5'-FAM represents a 5' FAM fluorophore conjugate group. 5'-AM-C6 refers to the amino C6 alkyl linker which is often used as an intermediate prior to conjugation to e.g. a GalNAc moiety. In compound 6, a mixed sequence gapmer oligonucleotide, wherein a small n is a DNA nucleoside, and a large N is a beta-D-oxy LNA nucleoside.

**Example 1: Synthesis of uncongugated, FAM-labeled and thiol-labeled oligonucleotides:**

Oligonucleotides were synthesized DMT-off using the phosphoramidite approach on a MerMade 12 or OligoPilot 100 synthesizer at 20 μmol or higher synthesis scales. Each oligonucleotide was cleaved and deprotected using aqueous ammonia for at least 5 hours at 60 °C. The crude compounds were purified by one of the following methods:

1) Ion-exchange HPLC, followed by ultrafiltration on Crossflow.
2) Direct ultrafiltration on Crossflow
3) Direct desalting using size exclusion chromatography

After purification the oligonucleotides were lyophilized. The purity and molecular weight of the oligonucleotides were characterized by UPLC-MS.

**Example 2: Synthesis of GalNAc-conjugated oligonucleotides:**

Oligonucleotides were synthesized as amino C6 at 5'-end using the phosphoramidite approach on MerMade 12 or OligoPilot 100 synthesizer at 20 μmol or higher synthesis scales. Each oligonucleo-tide was cleaved and deprotected using aqueous ammonia for at least 5 hours at 60 oC. The crude compounds were treated by one of the following methods:

1) Direct ultrafiltration on Crossflow
2) Dissolution in 0,1 M NaOH and evaporation
3) Precipitation in 2% LiClO4 in acetone, followed by evaporation of acetone.

The oligonucleotides were conjugated to a trivalent GalNAc conjugate using methods described in WO2014118267. The conjugated compounds were purified by one of the following methods:

1) Ion-exchange HPLC, followed by ultrafiltration on Crossflow.
2) Ion-exchange HPLC, followed by desalting using size exclusion chromatography After purification the oligonucleotides were lyophilized, spray dried or precipitated. The purity and molecular weight of the oligonucleotides were characterized by UPLC-MS.

The galNAc-C6linker conjugate moiety, as described in WO2014/118267 (conj 4a) is shown below:

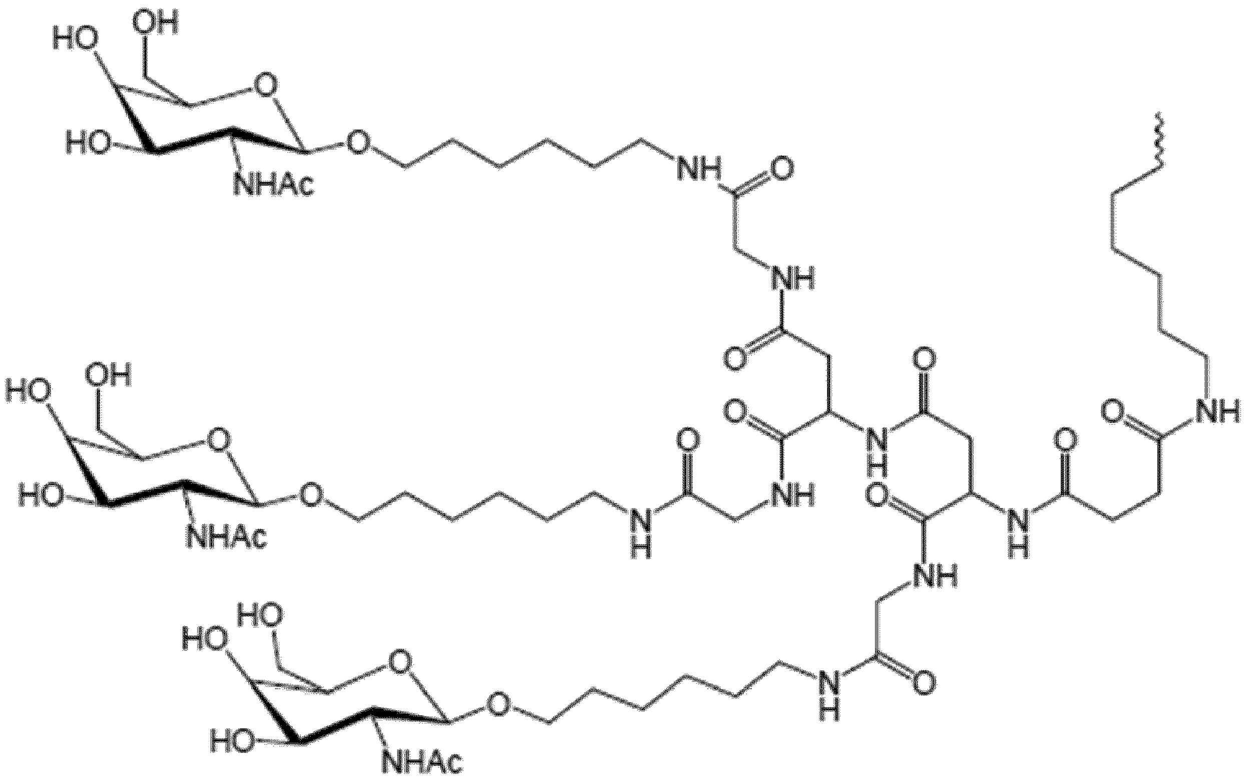

**Example 3: DVS Analysis**

DVS (Dynamic vapor sorption) analyses were done using automated gravimetric sorption systems. All samples were allowed to reach steady state at 0 % relative humidity (RH) inside the analytical instrument prior initiation of dynamic changes in humidity. The change in mass was analyzed both under increasing and decreasing RH conditions ranging from 0 to 90 or 95 % RH. Majority of the test compounds were analyzed using two cycles. One compound was only analyzed using one cycle. Two compounds were analyzed for two cycles, but due to technical problems with the instrument during measurements resulted in only one cycle was used.

The analyses were done in three laboratories using two different instruments:

1) The Surface Measurement Systems DVS instrument with DVS Data Analysis Suite
2) proUmid DVS instrument with SPS Software

The result output from both software types were dynamic change in samples mass as function of relative humidity conditions at fixed temperature.

Changes in relative humidity were done using different profiles in used instruments:

1) RH was increased in intervals: 0-5, 5-10, 10-20, 20-30, 30-40, 40-50, 50-60, 60-70, 70-80, 80-90, (90-95) and decreased again using the same intervals. The samples were allowed to equilibrate to conditions at each stage before changing the relative humidity to the next level. The scan rate corresponded to 1,5 to 9 % RH/h within the same compound depending on equilibration rate at current conditions.
2) Relative humidity was changed with fixed rate 5 % RH/h in the range 0-95 % RH.

The data obtained is shown in table 1 below. As illustrated in figure 1 the analyzed compounds showed hysteresis in change in mass during increasing and decreasing RH conditions. From the data no clear pattern or correlation between compounds was observed.

An average of all data points from all DVS analysis was used to study the link between change in mass in percent and the length of oligonucleotides.

**Table1:** Average mass change at different RH conditions.

| | Change in mass [%] | | | | | | | | |
|---|---|---|---|---|---|---|---|---|---|
| **Compound / RH %** | **10 %** | **20 %** | **30 %** | **40 %** | **50 %** | **60 %** | **70 %** | **80 %** | **90 %** |
| 1 | 0,049 | 0,0773 | 0,102 | 0,124 | 0,146 | 0,171 | 0,206 | 0,287 | 0,449 |
| 2 | 0,081 | 0,115 | 0,145 | 0,174 | 0,202 | 0,231 | 0,265 | 0,335 | 0,498 |
| 3 | 0,0463 | 0,0705 | 0,0946 | 0,116 | 0,138 | 0,161 | 0,195 | 0,27 | 0,42 |
| 4 | 0,0364 | 0,0588 | 0,0815 | 0,102 | 0,121 | 0,143 | 0,177 | 0,258 | 0,413 |
| 5 | 0,0469 | 0,0716 | 0,0942 | 0,115 | 0,135 | 0,158 | 0,192 | 0,267 | 0,418 |
| 6 | 0,0517 | 0,0785 | 0,1034 | 0,1258 | 0,1462 | 0,1708 | 0,2133 | 0,3111 | 0,5048 |
| 7 | 0,0376 | 0,069 | 0,0961 | 0,1225 | 0,1508 | 0,1836 | 0,223 | 0,2885 | 0,4421 |
| 8 | 0,0541 | 0,0866 | 0,1142 | 0,1413 | 0,1708 | 0,2052 | 0,248 | 0,3109 | 0,4149 |
| 9 | 0,0288 | 0,0449 | 0,0638 | 0,0846 | 0,1058 | 0,1288 | 0,1628 | 0,2465 | 0,4508 |
| 10 | 0,0535 | 0,0820 | 0,1094 | 0,1339 | 0,1617 | 0,1942 | 0,2362 | 0,3160 | 0,5415 |
| 11 | 0,0478 | 0,0767 | 0,1011 | 0,1244 | 0,1481 | 0,1775 | 0,2144 | 0,2715 | 0,4486 |
| 12 | 0,0520 | 0,0713 | 0,0913 | 0,1125 | 0,1333 | 0,1597 | 0,2084 | 0,3386 | 0,5238 |
| 13 | 0,0629 | 0,0832 | 0,1051 | 0,1293 | 0,1545 | 0,1849 | 0,2426 | 0,3886 | 0,5799 |
| 14 | 0,0402 | 0,0540 | 0,0765 | 0,1013 | 0,1281 | 0,1581 | 0,2046 | 0,3233 | 0,4933 |
| 15 | 0,0284 | 0,0464 | 0,0697 | 0,0960 | 0,1234 | 0,1540 | 0,1968 | 0,3171 | 0,5169 |
| 16 | 0,0623 | 0,0891 | 0,1180 | 0,1470 | 0,1759 | 0,2064 | 0,2608 | 0,4322 | 0,6722 |
| 17 | 0,0682 | 0,0877 | 0,1093 | 0,1331 | 0,1574 | 0,1869 | 0,2411 | 0,3811 | 0,6034 |
| 18 | 0,0488 | 0,0678 | 0,0911 | 0,1149 | 0,1392 | 0,1694 | 0,2066 | 0,3192 | 0,5086 |
| 19 | 0,0821 | 0,1057 | 0,1317 | 0,1619 | 0,1969 | 0,2405 | 0,3214 | 0,4882 | 0,7153 |
| 20 | 0,0692 | 0,0983 | 0,1284 | 0,1586 | 0,1901 | 0,2319 | 0,3318 | 0,4760 | 0,6264 |

By eye, the results shown in table 1 did not display any clear connection between RH conditions and change in mass (%). The percent wise change in mass was converted into weight of the water that is absorbed by a specific compound. The weight of water was furthermore converted to number of moles of water. The weight of compound was converted to number of moles of compound and the numbers of moles were multiplied by the number of nucleotides in each compound. The ratio was calculated between number of moles and the number of moles of nucleotides in each compound. The following equations were used to calculate number of water molecules per nucleotide in any given oligonucleotide sample - later referred to as Ratio. This step was necessary to standardize all results otherwise the weight gain in percent of different compounds from DVS analyses appeared random.

**Example 4 - Development of a mathematical algorithm for assessing the water content of oligonucleotides based upon DVS analysis.**

All the data from DVS analysis were converted using 4 equations.

Equations 1-3 showed reduction of unknown quantities using mathematical rules for isolations in order to get as few unknown factors as possible. Equation 4 was based on the results from equations 1-3 and is a combination of all 3 equations.

$\mathbf{m_{oligo}}$ = mass of a sample containing only oligonucleotide (no water).
$\mathbf{m_{water}}$ = mass of the water gained by the oligonucleotide after acclimatization.
$\mathbf{m_{oligo+water}}$ = mass of the oligonucleotide and water together which corresponds to $m_{oligo}$ + $m_{water}$.
$\mathbf{\Delta m\%}$ = change in mass in percent for a sample after acclimatization (written as e.g. 0,09 and not 9%). These data were provided by the DVS analyses.
$\mathbf{n_{oligo}}$= moles of a oligonucleotide

$n_{nucleotide}$= moles of all nucleotides =moles of oligonucleotides * number of nucleotides in the compound
$n_{water}$ = moles of water

Equation 1 was based on the assumption that if the DVS analysis of a given compound shows that this compound gains 20% weight after acclimatization, the new weight can be calculated:

$$m_{oligo} + 0{,}20 * m_{oligo}.$$

*Equation 1*

- $\boldsymbol{m_{oligo}} + \Delta \boldsymbol{m}\% * \boldsymbol{m_{oligo}} = \boldsymbol{m_{oligo+water}} \Leftrightarrow$
- $\boldsymbol{m_{oligo}}(\mathbf{1} + \Delta \boldsymbol{m}\%) = \boldsymbol{m_{oligo+water}} \Leftrightarrow$
- $\boldsymbol{m_{oligo}} = \frac{\boldsymbol{m_{oligo+water}}}{(\mathbf{1}+\Delta \boldsymbol{m}\%)}$

Equation 2 was a conversion of equation 1 to show that $m_{oligo}$ can be calculated using different factors. To be able to do that it was assumed that $m_{oligo+water} = m_{oligo} + m_{water}$.

*Equation 2*

- $\boldsymbol{m_{oligo}} = \frac{\boldsymbol{m_{oligo+water}}}{(\mathbf{1}+\Delta \boldsymbol{m}\%)} \Leftrightarrow$
- $\boldsymbol{m_{oligo}} = \frac{\boldsymbol{m_{oligo}}+\boldsymbol{m_{water}}}{(\mathbf{1}+\Delta \boldsymbol{m}\%)} \Leftrightarrow$
- $\boldsymbol{m_{oligo}} * (\mathbf{1} + \Delta \boldsymbol{m}\%) = \boldsymbol{m_{oligo}} + \boldsymbol{m_{water}} \Leftrightarrow$
- $\boldsymbol{m_{oligo}} * (\mathbf{1} + \Delta \boldsymbol{m}\%) - \boldsymbol{m_{oligo}} = \boldsymbol{m_{water}} \Leftrightarrow$
- $\boldsymbol{m_{oligo}} + \boldsymbol{m_{oligo}} * \Delta \boldsymbol{m}\% - \boldsymbol{m_{oligo}} = \boldsymbol{m_{water}} \Leftrightarrow$
- $\boldsymbol{m_{oligo}} * \Delta \boldsymbol{m}\% = \boldsymbol{m_{water}}$

Equation 3 was combination of equation 1 and 2. $m_{oligo}$ was isolated in both equation 1 and 2 and joined together to form equation 3.

*Equation 3*

- $\frac{\boldsymbol{m_{water}}}{\Delta \boldsymbol{m}\%} = \boldsymbol{m_{oligo}} = \frac{\boldsymbol{m_{oligo+water}}}{(\mathbf{1}+\Delta \boldsymbol{m}\%)} \Leftrightarrow$
- $\boldsymbol{m_{water}} = \frac{\boldsymbol{m_{oligo+water}} * \Delta \boldsymbol{m}\%}{(\mathbf{1}+\Delta \boldsymbol{m}\%)}$

Equation 4 was made using equations 1,2 and 3 together with formula m=M*n. By combining all formulas it was possible reduce the number of unknown factors to just one (Δm%).

*Equation 4*

- $n_{nucleotides} = n_{oligo} * no\ nucleotides$
- $Ratio = \frac{n_{water}}{n_{nucleotides}} \Leftrightarrow$
- $Ratio = \frac{m_{water}}{M_{water}} / \frac{m_{oligo} * no\ nucleotides}{M_{oligo}} \Leftrightarrow$
- $Ratio = \frac{m_{oligo} * \Delta m\%}{M_{water}} / \frac{m_{oligo} * no\ nucleotides}{M_{oligo}} \Leftrightarrow$
- $Ratio = \frac{\Delta m\% * M_{oligo}}{M_{water} * no\ nucleotides}$

**Example 5 - Application of ration model to the DVS raw data**

The ratio was calculated for all tested compounds and at all RH datapoint using the equation 4 and the average change in mass for each compound. $\Delta$m% data were obtained from table with overview of mass change for each compound at different room humidity. The other factors such as $M_{oligo}$ and number of nucleotides are compound specific and can be calculated while $M_{water}$ is always constant 18,02 g/mol. The results are summarized in the table 2 below.

**Table2:** Ratio of water molecules is calculated using Equation 4

| | **Ratio (water molecules per nucleotide)** | | | | | | | | |
|---|---|---|---|---|---|---|---|---|---|
| **Compound / RH %** | **10 %** | **20 %** | **30 %** | **40 %** | **50 %** | **60 %** | **70 %** | **80 %** | **90 %** |
| 1 | 1,3 | 2,0 | 2,6 | 3,2 | 3,8 | 4,4 | 5,3 | 7,4 | 11,6 |
| 2 | 1,5 | 2,2 | 2,8 | 3,3 | 3,8 | 4,4 | 5,1 | 6,4 | 9,5 |
| 3 | 1,2 | 1,8 | 2,4 | 3,0 | 3,5 | 4,1 | 5,0 | 6,9 | 10,7 |
| 4 | 0,9 | 1,5 | 2,1 | 2,6 | 3,1 | 3,7 | 4,6 | 6,7 | 10,6 |
| 5 | 1,2 | 1,9 | 2,4 | 3,0 | 3,5 | 4,1 | 5,0 | 6,9 | 10,8 |
| 6 | 1,2 | 1,9 | 2,5 | 3,0 | 3,5 | 4,1 | 5,1 | 7,5 | 12,2 |
| 7 | 0,7 | 1,3 | 1,9 | 2,4 | 2,9 | 3,5 | 4,3 | 5,6 | 8,5 |
| 8 | 1,1 | 1,7 | 2,3 | 2,8 | 3,4 | 4,1 | 4,9 | 6,2 | 8,2 |
| 9 | 0,7 | 1,2 | 1,6 | 2,2 | 2,7 | 3,3 | 4,2 | 6,4 | 11,6 |
| 10 | 1,1 | 1,6 | 2,2 | 2,7 | 3,2 | 3,9 | 4,7 | 6,3 | 10,8 |
| 11 | 1,0 | 1,5 | 2,0 | 2,5 | 3,0 | 3,5 | 4,3 | 5,4 | 9,0 |
| 12 | 1,3 | 1,8 | 2,3 | 2,9 | 3,4 | 4,1 | 5,3 | 8,6 | 13,3 |
| 13 | 1,2 | 1,6 | 2,0 | 2,5 | 3,0 | 3,5 | 4,7 | 7,5 | 11,1 |
| 14 | 0,8 | 1,1 | 1,5 | 2,0 | 2,5 | 3,1 | 4,0 | 6,3 | 9,6 |
| 15 | 0,6 | 1,0 | 1,5 | 2,1 | 2,6 | 3,3 | 4,2 | 6,8 | 11,0 |
| 16 | 1,3 | 1,8 | 2,4 | 3,0 | 3,6 | 4,2 | 5,3 | 8,7 | 13,6 |
| 17 | 1,6 | 2,1 | 2,6 | 3,2 | 3,8 | 4,5 | 5,8 | 9,1 | 14,4 |
| 18 | 0,9 | 1,3 | 1,8 | 2,2 | 2,7 | 3,3 | 4,0 | 6,1 | 9,8 |
| 19 | 1,5 | 2,0 | 2,5 | 3,0 | 3,7 | 4,5 | 6,0 | 9,1 | 13,4 |
| 20 | 1,5 | 2,1 | 2,8 | 3,4 | 4,1 | 5,0 | 7,2 | 10,3 | 13,5 |
| Average | 1,1 | 1,7 | 2,2 | 2,8 | 3,3 | 3,9 | 5,0 | 7,2 | 11,2 |

It is important to remember that oligonucleotides consist of a series of nucleotides and the number varies

between compounds. It is also important to distinguish between change in mass in percent (introduced in part 1) and room humidity in percent (introduced in part 2).

**Example 6 - Comparative analysis of the ratio data**

When all average results from DVS analysis were combined in a graph it was possible visualize a trend for the behavior between all compounds in the interval 10-70 % RH. In this interval, all compounds showed a linear correlation between relative humidity and number of water molecules absorbed per nucleotide in the compound. This shows that all tested compounds had very similar behavior (Figure 2), something which was not transparent or predicted from the original DVS analysis data (table 1).

The tested compounds are very different in design and still show very similar behavior and the average number of water molecules per nucleotide was proved to be very similar between all tested compounds. Assuming that all untested oligonucleotides behave in the same manor a model can be built on already analyzed compounds in order to predict behavior of other oligonucleotides, based only on knowledge of their length (or MW) of the oligonucleotide (part), their weight, and the RH of the atmosphere - notably:

For both LNA and 2-MOE modified oligonucleotides, and for oligonucleotide comprising only phosphorothioate or both phosphorothioate and phosphodiester linkages, there is a remarkable close correlation between the hydration level of the oligonucleotide and the relative humidity of the atmosphere. Furthermore the addition of a conjugate group, such as a GalNAc conjugate, which presents a large number of potential hydrogen bonding sites with water, did not notably affect the ratio model, illustrating that surprisingly the combined use of DVS analysis and the ratio model developed by the inventors is suitable for use with sugar modified oligonucleotides, linkage modified oligonucleotides, and oligonucleotide conjugates.

By combining the results from the following graph in the relative humidity interval 10-70 % it is possible to draw a trendline also called a Ratio-model (Figure 2 & 7).

Based on the Ratio-model it is now possible to calculate number of water molecules per nucleotide at any humidity conditions between 10-70 % for new compounds that had not been analyzed using DVS analysis before. An schematic overview of the use of the ratio model is shown in figure 3.

If information about ratio of water molecules per nucleotide is combined with knowledge about length and molecular weight of the new compound, a theoretical percent wise content of water can be calculated for any oligonucleotide powder using Equation 4.

**Example 7: Ratio model - Average versus maximum or minimum**

Ratio-model mentioned before was based on the average results from DVS analysis. Each DVS analysis are also based on the average results since all compound showed hysteresis in change in mass during increasing and decreasing RH conditions meaning all compounds behaved differently during sorption and desorption of water.

To investigate how precise is using the Ratio-model based on average water absorption for formulation oligonucleotides the following calculations were done. A Ration-model for maximum and a Ratio-model for minimum were made using the highest and lowest results from all obtained DVS analyses.

3 trend lines were made based on the following data points for each compound in the interval 10-60/70 % relative humidity. All trend lines showed correlation coefficient close to 1. These trend lines were named Ratio model min, max and average.

*Ratio model average:* Shows average of all available data points converted from DVS analysis in the interval 10-70%. Results from absorption and sorption were included here despite the fact they display a hysteresis.

*Ratio model max:* The maximum data points were found for each value of RH in the interval 10-60%. The values did not originate from the same compound throughout the interval. The data point for 70 % RH was excluded due to abnormal behavior.

*Ratio model min:* The minimum data points were found for each value of RH in the interval 10-70%. The values did not originate from the same compound throughout the interval.

Figure 4: The graph shows trend lines based on average, maximum and minimum values from the converted DVS analysis. 70 % RH for maximum values is excluded from this model.

**Example 8: Formulation of an oligonucleotide solution based upon the DVS ratio model:**

To assess the deviation involved in this approximation between maximum and minimum comparing to the average model the following theoretical experiment was done.

**Average change in mass**

The change of mass can be calculated for any unknown compound using the Ratio-model in combination with equation 4. For high throughput formulation of oligonucleotides an average Ratio-model is suitable.

$$\text{Ratio} = 0{,}061 * RH + 0{,}4218$$

$$\Delta\text{m\%} = \frac{Ratio * MW_{water} * no\ nucleotides}{MW_{compound,\ Na-salt}}$$

Ratio = number of water molecules absorbed per nucleotide in the compound (GalNAc excluded from this).
RH = current room humidity that compounds are acclimatized to.
Δm% = change in mass of the compound after acclimatization.
MW(water) = 18,02 g/mol, molecular weight of water.
MW(compound, Na-salt) = molecular weight of the compound as sodium salt.
No nucleotides = number of nucleotides in the compound (if compound contains GalNAc, it should not be included in this number).

2 mg/ml, 5 ml of Compound A is needed.
Theoretical mass needed for this solution: 2 mg/ml * 5 ml = 10 mg
Compound A information: MW(Na-salt)=4900 g/mol, compound contains 14 nucleotides

RH: 45 %

$$\text{Ratio} = 0{,}061 * RH + 0{,}4218 = 0{,}061 * 45 + 0{,}4218 = 3{,}17$$

$$\Delta\text{m\%} = \frac{Ratio * MW_{water} * no\ nucleotides}{MW_{compound\ A,\ Na-salt}} = \frac{3{,}17 * 18{,}02 g/mol * 14\ nucleotides}{4900\ g/mol} = 0{,}16$$

Assuming that the actual weight can be calculated:

$$m_{compound+water} = m_{compound} + \Delta\text{m\%} * m_{compound} = 10\ mg + 0{,}16 * 10\ mg = 11{,}6\ mg$$

Resulting in 11.6 mg of compound A needs to be weight out in order to get 5 ml of 2 mg/ml solution.

**Example 9: Error margin calculation**

To investigate how different are the calculation based on the average compared to the calculation based on the outermost results the following calculations were done using the 3 Ratio-model from above. By using Ratio-models for maximum, average and minimum followed by using these results in Equation 4 it is possible to calculate the difference in mass and hence deviation in percent.

All the following calculations are based on the same solution of compound A from the example above. First step was to calculate the ratio of water molecules per nucleotide in compound A using Ratio-models for maximum, average and minimum. The three formulas were taken from the graph above.

Max: Ratio = 0,0711 $*$ $RH$ + 1,365 = 0,0711 $*$ 45 + 1,365 = 4,56
Average: Ratio = 0,061 $*$ $RH$ + 0,4218 = 0,061 $*$ 45 + 0,4218 = 3,17
Min: Ratio = 0,0545 $*$ $RH$ - 0,6818 = 0,0545 $*$ 45 - 0,6818 = 1,77

The ratios were used in Equation 4 to calculate the theoretical increase in weight for compound A for RH at 45% as mentioned above.

Maximum expected change in mass:

$$\Delta m\% = \frac{Ratio * MW_{water} * no\ nucleotides}{MW_{compound\ A,\ Na-salt}} = \frac{4{,}56 * 18{,}02 g/mol * 14\ nucleotides}{4900\ g/mol} = 0{,}23$$

Average expected change in mass:

$$\Delta m\% = \frac{Ratio * MW_{water} * no\ nucleotides}{MW_{compound\ A,\ Na-salt}} = \frac{3{,}17 * 18{,}02 g/mol * 14\ nucleotides}{4900\ g/mol} = 0{,}16$$

Minimum expected change in mass:

$$\Delta m\% = \frac{Ratio * MW_{water} * no\ nucleotides}{MW_{compound\ A,\ Na-salt}} = \frac{1{,}77 * 18{,}02 g/mol * 14\ nucleotides}{4900\ g/mol} = 0{,}09$$

When the percentwise changes in mass were calculated, the actual weight of a sample could also be calculated.

Maximum model:

$$m_{compound+water} = m_{compound} + \Delta m\% * m_{compound} = 10\ mg + 0{,}23 * 10\ mg = 12{,}3\ mg$$

Average model:

$$m_{compound+water} = m_{compound} + \Delta m\% * m_{compound} = 10\ mg + 0{,}16 * 10\ mg = 11{,}6\ mg$$

Minimum model:

$$m_{compound+water} = m_{compound} + \Delta m\% * m_{compound} = 10\ mg + 0{,}09 * 10\ mg = 10{,}9\ mg$$

The purpose was to compare the outer most results to the average therefor the difference between maximum vs average models and minimum vs average models were calculated:

$$\left|\frac{m_{average\ model} - m_{maximum\ model}}{m_{average\ model}} * 100\%\right| = \left|\frac{11{,}6 - 12{,}3}{11{,}6} * 100\%\right| = 6\%$$

$$\frac{m_{average\ model} - m_{minimum\ model}}{m_{average\ model}} * 100\% = \frac{11{,}6 - 10{,}9}{11{,}6} * 100\% = 6\%$$

The difference for needed compound between maximum vs average models and minimum vs average is only 6 %.

This means that despite all analyzed compounds showed hysteresis in change in mass during increasing and decreasing RH conditions the deviation introduced by the average model is very low. Even if the unknown compound is a borderline case and have values close to the maximum or minimum, the highest expected deviation from the average model is 6%. Despite this deviation, the Ratio-model based on the average results is better than a method relying on a UV measurement and theoretically calculated extinction coefficients based method which may give even higher deviations.

**Example 10 - Comparison with alternative formulation methods**

UV measurements are commonly accepted as a one of the standard methods for concentration determination

in solutions with oligonucleotide. To show that concentrations delivered by using Ratio-model are very close to the results delivered by UV measurement and experimentally measured extinction coefficients the following experiment was done.

The first step was to determine extinction coefficients experimentally for series of oligonucleotides. A theoretical extinction coefficient was also found for these compounds. The second step was formulation of solutions for the same compounds using Ratio-model (based on average results). Concentration was calculated in the solutions using Beer-Lambert law and the two available extinction coefficients for each compound.

**Experimental determination of extinction coefficient**

Water content was analyzed in the test compounds using TGA analysis, and the results were used in the next step.

The test compounds were weighted out and the weight was corrected for water content. All the samples were diluted to 0,05 mg/ml with water, followed by further dilution to 0,033 mg/ml, 0,025 mg/ml, 0,0042 mg/ml. The absorbance was measured at 260 nm and plotted in a graph with concentration X-axis and absorbance on Y-axis. A trend line was made for each compound and the slopes were the extinction coefficients. The results were also corrected for the purity of the compounds.

**Comparison of formulation methods**

Compounds with available experimentally measured extinction coefficients were allowed to acclimatize for at least 4 hours in the suction cabinet where the weighing procedure was done. The room humidity reader was also placed in that suction cabinet. The room humidity was read and theoretical water content was calculated using the equation based on the average results. Each compound was weight out twice, the powder was dissolved in corresponding amount of water to result 2 mg/ml solutions. Absorbance was measured on an Eppendorf Biophotometer in each solution. Concentrations were calculated using Beer-Lambert law using both the theoretical and the measured extinction coefficients for test compounds. The results are summarized in the table 3 below.

**Table 3:**

| | | | | | Theoretical e260 | | Measured e260 | |
|---|---|---|---|---|---|---|---|---|
| Compound | Dilution | Abs | MW (Na-salt) g/mol | Conc. based on Ratio-model mg/ml | e260 | Calculated conc. mg/ml | e260 | Calculate d conc. mg/ml |
| 3 | 100 | 0,358 | 7337,8 | 2 | 167,3 | 1,57 | 145,6 | 1,80 |
| 3 | 100 | 0,386 | 7337,8 | 2 | 167,3 | 1,69 | 145,6 | 1,95 |
| 8 | 100 | 0,372 | 7134,2 | 2 | 171,5 | 1,55 | 132,4 | 2,00 |
| 8 | 100 | 0,366 | 7134,2 | 2 | 171,5 | 1,52 | 132,4 | 1,97 |
| 9 | 100 | 0,391 | 6967,7 | 2 | 143,8 | 1,89 | 131 | 2,08 |
| 9 | 100 | 0,381 | 6967,7 | 2 | 143,8 | 1,85 | 131 | 2,03 |
| 17 | 100 | 0,391 | 9061,1 | 2 | 221,7 | 1,60 | 192,7 | 1,84 |
| 17 | 100 | 0,406 | 9061,1 | 2 | 221,7 | 1,66 | 192,7 | 1,91 |

There is much more concordance between the concentration based on the Ratio-model and the concentrations based on the measured extinction coefficients. The results based on the theoretical extinction coefficients give large difference from the other two methods that are usually too low. This means that the same compounds would have been dosed in to high concentrations if concentrations in test solutions were adjusted using theoretical extinction coefficients.

To illustrate the difference between methods the results were collected in one graph (figure 5).

Visually there was good correlation between the blue and green bars representing concentrations based on the Ratio-model and measured extinction coefficients. To show that the difference is between these two methods is below 10 %, error bars were added to the concentrations based on the Ratio-model to represent +/- 10 % of the target concentration. The results based the theoretical calculated extinction coefficient gave results lie outside the 10% majority of the time.

This experiment shows that there is a very good correlation between the Ratio-model and one of the most acknowledged and widely used formulation method - absorbance measurements using measured extinction coefficients. Therefore the concentration determination by absorbance measurement can be replaced by preparation of solutions using Ratio-model instead.

The proposed method has also the advantage of fewer pipetting steps compared to absorbance measurements, which can be source of error.

**Example 11** - Detailed investigation of absorption and sorption rates

The samples were analyzed using the method described in Example 3: DVS Analysis (page 39). Software option 1 and changes in relative humidity were done using profile 1 for both analyses.

Table 4

| **Compound 2** | (figure 6) | **Compound 5** | GalNAc |
|---|---|---|---|
| Interval RH | Aclimatization time cycle A (hours) | Interval RH | Aclimatization time cycle B (hours) |
| 10-20 | 1,5 | 5-10 | 1,2 |
| 20-30 | 2,1 | 10-20 | 1,5 |
| 30-40 | 2,1 | 20-30 | 1,5 |
| 40-50 | 2,0 | 30-40 | 1,8 |
| 50-60 | 1,8 | 40-50 | 1,9 |
| 60-70 | 1,5 | 50-60 | 1,6 |
| 70-80 | 1,2 | 60-70 | 1,2 |
| 80-90 | 1,7 | 70-80 | 2,0 |
| 90-95 | 7,4 | 80-90 | 2,2 |
| 95-90 | 8,5 | 90-80 | 4,0 |
| 90-80 | 4,6 | 80-70 | 3,4 |
| 80-70 | 3,4 | 70-60 | 2,8 |
| 70-60 | 2,6 | 60-50 | 2,6 |
| 60-50 | 3,0 | 50-40 | 2,7 |
| 50-40 | 3,6 | 40-30 | 3,3 |
| 40-30 | 3,9 | 30-20 | 4,0 |
| 30-20 | 3,7 | 20-10 | 5,7 |
| 20-10 | 3,6 | 10-5 | 6,0 |

## Claims

1. A method for determining the dry weight of an oligonucleotide salt ($m_{compound\ salt}$) present in a hydrated powdered form of an oligonucleotide salt, said method comprising:

   a. Obtaining a solid powdered form of the oligonucleotide compound salt of known molecular weight ($MW_{compound\ salt}$);
   b. Expose the solid powdered form of the oligonucleotide compound salt to an atmosphere for a period of at

least 2 hours, wherein the atmosphere has a relative humidity of between about 10 - about 70%;
c. Measure the weight of the solid powdered form of the oligonucleotide compound salt after said period of at least 2 hours (W = $m_{compound\ salt}$+ $H_2O$);
d. Determine the dry weight of the oligonucleotide

wherein the dry weight ($m_{compound\ salt}$) is determined by the formula:

$$m_{compound\ salt} = \frac{W}{1 + \frac{Y\ x\ MW_{H_2O}\ x\ Z}{MW_{compound\ salt}}}$$

wherein

*W* is the weight of the solid powdered form of the oligonucleotide salt measured in step c;
*Y* is 0.06±0.011, such as Y is a value between 0.0545 and 0.0711;
$MW_{H2O}$ is the molecular weight of water;
Z is the number of nucleotides in the oligonucleotide;
$MW_{compound\ salt}$ is the molecular weight of the oligonucleotide salt.

**2.** A method for determining the number of moles of an oligonucleotide salt ($n_{compound\ salt}$) present in a hydrated powdered form of an oligonucleotide salt, said method comprising performing the method of claim 1, wherein the number of moles is determined by the formula:

$$n_{compound\ salt} = m_{compound\ salt} / MW_{compound\ salt}$$

**3.** A method for preparing a solvent formulation of an oligonucleotide salt with a defined concentration ($m_{compound\ salt}$ /unit volume), said method comprising performing the method according to claim 1, and subsequently dissolving a calculated dry weight of the oligonucleotide salt in a known volume of solvent to provide the solvent formulation of an oligonucleotide salt with defined concentration (m *compound* $_{salt}$ /unit volume).

**4.** A method for preparing a solvent formulation of an oligonucleotide salt with defined molar concentration ($n_{compound\ salt}$ /unit volume), said method comprising performing the method according to claim 2, and subsequently dissolving a calculated number of moles of the oligonucleotide salt in a known volume of solvent to provide the solvent formulation of an oligonucleotide salt with defined molar concentration n $_{compound\ salt}$ /unit volume).

**5.** The method according to claims 3 or 4, wherein said method further comprises the step of aliquoting the solvent formulation into a vial or syringe.

**6.** The method according to claim 5, wherein the aliqoting in to the vial or syringe is aliquoting of a unit dose form of the oligonucleotide salt.

**7.** The method according to any one of claims 3-6, wherein the method further comprises the step of sterilizing the solvent formulation (can be done prior to, during or after aliquoting according to claim 5 or 6).

**8.** The method according to any one of claims 5 or 6, wherein said method further comprises the step of aliquoting the solvent formulation into a vial, followed by the step of removing the solvent from the solvent composition to provide a vial containing a known quantity of dry oligonucleotide salt.

**9.** The method according to any one of claims 1-8, wherein the oligonucleotide is an oligonucleotide conjugate.

**10.** The method according to claim 9, wherein the oligonucleotide conjugate comprises a carbohydrate conjugate moiety, such as a GalNAc conjugate moiety, or a reporter group, such as a fluorescent group (e.g. FAM).

**11.** The method according to any one of claims 1 - 10, wherein the atmosphere of step b. has a known relative humidity of between about 10 - about 60%, or a relative humidity of between about 20 - about 50%.

**12.** The method according to any one of claims 1 - 11, wherein the period referred to in step b. is at least about 2 hours or at least about 3 hours or at least about 4 hours.

**13.** The method according to any one of claims 1 - 12, wherein the oligonucleotide comprises one or more nucleosides selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, 2'-F-ANA nucleoside, and a LNA nucleoside(s).

**14.** The method according to any one of claims 1 - 13, wherein the oligonucleotide comprises phosphorothioate internucleoside linkages.

**15.** The method according to any one of claims 1 - 14, wherein the ionic cation of the salt is selected from a metal cation, such as a sodium or potassium cation, or an ammonium cation.

**16.** The method according to any one of claims 1 - 15, wherein the oligonucleotide has a length of 8 - 25 contiguous nucleotides, such as 12 - 20 nucleotides.

## Patentansprüche

**1.** Verfahren zum Bestimmen des Trockengewichtes eines Oligonukleotidsalzes ($m_{Verbindungssalz}$), das in einer hydratisierten pulverisierten Form eines Oligonukleotidsalzes vorliegt, wobei das Verfahren Folgendes umfasst:

a. Erhalten einer festen pulverisierten Form des Oligonukleotidverbindungssalzes mit bekanntem Molekulargewicht ($MW_{Verbindungssalz}$);
b. Aussetzen der festen pulverisierten Form des Oligonukleotidverbindungssalzes einer Atmosphäre für einen Zeitraum von mindestens 2 Stunden, wobei die Atmosphäre eine relative Feuchte zwischen etwa 10 und etwa 70 % hat;
c. Messen des Gewichtes der festen pulverisierten Form des Oligonukleotidverbindungssalzes nach diesem Zeitraum von mindestens 2 Stunden (W = $m_{Verbindungssalz}$ + $H_2O$);
d. Bestimmen des Trockengewichtes des Oligonukleotids,

wobei das Trockengewicht ($m_{Verbindungssalz}$) mit der folgenden Formel bestimmt wird:

$$m_{Verbindungssalz} = \frac{W}{1 + \frac{Y \, x \, MW_{H_2O} \, x \, Z}{MW_{Verbindungssalz}}}$$

wobei

$W$ das Gewicht der festen pulverisierten Form des Oligonukleotidsalzes ist, das in Schritt c gemessen wurde;
$Y$ 0,06 $\pm$ 0,011 ist, beispielsweise ist $Y$ ein Wert zwischen 0,0545 und 0,0711;
$MW_{H2O}$ das Molekulargewicht von Wasser ist;
Z die Anzahl an Nukleotiden in dem Oligonukleotid ist;
$MW_{Verbindungssalz}$ das Molekulargewicht des Oligonukleotidsalzes ist.

**2.** Verfahren zum Bestimmen der Molzahl eines Oligonukleotidsalzes ($n_{Verbindungssalz}$), das in einer hydratisierten pulverisierten Form eines Oligonukleotidsalzes vorliegt, wobei das Verfahren das Durchführen des Verfahrens von Anspruch 1 umfasst, wobei die Molzahl mit der folgenden Formel bestimmt wird:

$$n_{Verbindungssalz} = m_{Verbindungssalz} / MW_{Verbindungssalz}$$

**3.** Verfahren zum Herstellen einer Lösungsmittelformulierung von einem Oligonukleotidsalz mit einer definierten Konzentration ($m_{VerbindungssaLz}$/Volumeneinheit), wobei das Verfahren das Durchführen des Verfahrens nach Anspruch 1 und anschließend das Auflösen eines berechneten Trockengewichtes des Oligonukleotidsalzes in einem bekannten Lösungsmittelvolumen, um die Lösungsmittelformulierung eines Oligonukleotidsalzes mit definierter Konzentration ($m_{Verbindungssalz}$/Volumeneinheit) bereitzustellen, umfasst.

**4.** Verfahren zum Herstellen einer Lösungsmittelformulierung von einem Oligonukleotidsalz mit einer definierten Molarität ($n_{Verbindungssalz}$/Volumeneinheit), wobei das Verfahren das Durchführen des Verfahrens nach Anspruch 2 und anschließend das Auflösen einer berechneten Molzahl des Oligonukleotidsalzes in einem bekannten Lösungsmittelvolumen, um die Lösungsmittelformulierung eines Oligonukleotidsalzes mit definierter Molarität ($n_{Verbindungssalz}$/Volumeneinheit) bereitzustellen, umfasst.

**5.** Verfahren nach den Ansprüchen 3 oder 4, wobei das Verfahren ferner den Schritt des Aliquotierens der Lösungsmittelformulierung in ein Fläschchen oder eine Spritze umfasst.

**6.** Verfahren nach Anspruch 5, wobei das Aliquotieren in das Fläschchen oder die Spritze das Aliquotieren einer Einheitsdosisform des Oligonukleotidsalzes ist.

**7.** Verfahren nach einem der Ansprüche 3 - 6, wobei das Verfahren ferner den Schritt des Sterilisierens der Lösungsmittelformulierung umfasst (kann vor, während oder nach dem Aliquotieren nach Anspruch 5 oder 6 erfolgen).

**8.** Verfahren nach einem der Ansprüche 5 oder 6, wobei das Verfahren ferner den Schritt des Aliquotierens der Lösungsmittelformulierung in ein Fläschchen umfasst, gefolgt von dem Schritt des Entfernens des Lösungsmittels aus der Lösungsmittelzusammensetzung, um ein Fläschchen bereitzustellen, das eine bekannte Menge an trockenem Oligonukleotidsalz enthält.

**9.** Verfahren nach einem der Ansprüche 1 - 8, wobei das Oligonukleotid ein Oligonukleotidkonjugat ist.

**10.** Verfahren nach Anspruch 9, wobei das Oligonukleotidkonjugat eine Kohlenhydratkonjugateinheit, wie eine GalNAc-Konjugateinheit, oder eine Reportergruppe, wie eine fluoreszierende Gruppe (z. B. FAM), umfasst.

**11.** Verfahren nach einem der Ansprüche 1 - 10, wobei die Atmosphäre von Schritt b. eine bekannte relative Feuchte zwischen etwa 10 und etwa 60 % oder eine relative Feuchte zwischen etwa 20 und etwa 50 % hat.

**12.** Verfahren nach einem der Ansprüche 1 - 11, wobei der Zeitraum, auf den in Schritt b. Bezug genommen wird, mindestens etwa 2 Stunden oder mindestens etwa 3 Stunden oder mindestens etwa 4 Stunden beträgt.

**13.** Verfahren nach einem der Ansprüche 1 - 12, wobei das Oligonukleotid ein oder mehrere Nukleosid(e) umfasst, das/die aus der Gruppe ausgewählt ist/sind, die aus 2'-O-Alkyl-RNA, 2'-O-Methyl-RNA, 2'-Alkoxy-RNA, 2'-O-Methoxyethyl-RNA (MOE), 2'-Amino-DNA, 2'-Fluor-RNA, 2'-F-ANA-Nukleosid und LNA-Nukleosid(en) besteht.

**14.** Verfahren nach einem der Ansprüche 1 - 13, wobei das Oligonukleotid Phosphorothioat-Internukleosid-Verknüpfungen umfasst.

**15.** Verfahren nach einem der Ansprüche 1 - 14, wobei das ionische Kation des Salzes aus einem Metallkation, wie einem Natrium- oder Kaliumkation, oder einem Ammoniumkation ausgewählt ist.

**16.** Verfahren nach einem der Ansprüche 1 - 15, wobei das Oligonukleotid eine Länge von 8 - 25 aufeinanderfolgenden Nukleotiden, wie 12 - 20 Nukleotiden hat.

## Revendications

**1.** Procédé de détermination du poids sec d'un sel oligonucléotidique ($m_{sel\ de\ composé}$) présent sous une forme de poudre hydratée d'un sel oligonucléotidique, ledit procédé comprenant :

a. l'obtention d'une forme de poudre solide du sel de composé oligonucléotidique d'un poids moléculaire ($MW_{sel\ de\ composé}$) connu ;
b. l'exposition de la forme de poudre solide du sel de composé oligonucléotidique à une atmosphère pendant une période d'au moins 2 heures, dans lequel l'atmosphère a une humidité relative comprise entre environ 10 et environ 70 % ;
c. la mesure du poids de la forme de poudre solide du sel de composé oligonucléotidique après ladite période d'au moins 2 heures (W = $m_{sel\ de\ composé}$ + $H_2O$) ;
d. la détermination du poids sec de l'oligonucléotide

dans lequel le poids sec ($m_{sel\ de\ composé}$) est déterminé par la formule :

$$m_{sel\ de\ composé} = \frac{W}{1 + \frac{Y\ x\ MW_{H_2O}\ x\ Z}{MW_{sel\ de\ composé}}}$$

dans laquelle

*W* représente le poids de la forme de poudre solide du sel oligonucléotidique mesuré à l'étape c ;
*Y* représente 0,06 ± 0,011, tel que Y représente une valeur entre 0,0545 et 0,0711 ;
$MW_{H2O}$ représente le poids moléculaire de l'eau ;
Z représente le nombre de nucléotides dans l'oligonucléotide ;
$MW_{sel\ de\ composé}$ représente le poids moléculaire du sel oligonucléotidique.

**2.** Procédé de détermination du nombre de moles d'un sel oligonucléotidique ($n_{sel\ de\ composé}$) présent sous une forme de poudre hydratée d'un sel oligonucléotidique, ledit procédé comprenant la mise en oeuvre du procédé selon la revendication 1, dans lequel le nombre de moles est déterminé par la formule :

$$n_{\ sel\ de\ composé} = m_{\ sel\ de\ composé} / MW_{\ sel\ de\ composé}$$

**3.** Procédé de préparation d'une formulation à base de solvant d'un sel oligonucléotidique ayant une concentration ($m_{sel\ de\ composé}$ /unité de volume) définie, ledit procédé comprenant la mise en oeuvre du procédé selon la revendication 1, et ensuite la dissolution d'un poids sec calculé du sel oligonucléotidique dans un volume connu de solvant pour fournir la formulation à base de solvant d'un sel oligonucléotidique ayant une concentration ($m_{sel\ de\ composé}$ /unité de volume) définie.

**4.** Procédé de préparation d'une formulation à base de solvant d'un sel oligonucléotidique ayant une concentration molaire ($n_{sel\ de\ composé}$ /unité de volume) définie, ledit procédé comprenant la mise en oeuvre du procédé selon la revendication 2, et ensuite la dissolution d'un nombre de moles calculé du sel oligonucléotidique dans un volume connu de solvant pour fournir la formulation à base de solvant d'un sel oligonucléotidique ayant une concentration molaire ($n_{sel\ de\ composé}$ /unité de volume) définie.

**5.** Procédé selon les revendications 3 ou 4, dans lequel ledit procédé comprend en outre l'étape d'aliquotage de la formulation à base de solvant dans un flacon ou une seringue.

**6.** Procédé selon la revendication 5, dans lequel laliquotage dans le flacon ou la seringue est l'aliquotage d'une forme galénique unitaire du sel oligonucléotidique.

**7.** Procédé selon l'une quelconque des revendications 3 à 6, dans lequel le procédé comprend en outre l'étape de stérilisation de la formulation à base de solvant (pouvant être réalisée avant, pendant ou après l'aliquotage selon la revendication 5 ou 6).

**8.** Procédé selon l'une quelconque des revendications 5 ou 6, dans lequel ledit procédé comprend en outre l'étape d'aliquotage de la formulation à base de solvant dans un flacon, suivie par l'étape d'élimination du solvant de la composition de solvant pour fournir un flacon contenant une quantité connue de sel oligonucléotidique sec.

**9.** Procédé selon l'une quelconque des revendications 1 à 8, dans lequel l'oligonucléotide est un conjugué oligonucléotidique.

**10.** Procédé selon la revendication 9, dans lequel le conjugué oligonucléotidique comprend une fraction de conjugué glucidique, telle qu'une fraction de conjugué de GalNAc, ou un groupe rapporteur, tel qu'un groupe fluorescent (par exemple FAM).

**11.** Procédé selon l'une quelconque des revendications 1 à 10, dans lequel l'atmosphère de l'étape b. a une humidité relative connue comprise entre environ 10 et environ 60 %, ou une humidité relative comprise entre environ 20 et environ 50 %.

**12.** Procédé selon l'une quelconque des revendications 1 à 11, dans lequel la période décrite à l'étape b. est d'au moins environ 2 heures ou d'au moins environ 3 heures ou d'au moins environ 4 heures.

**13.** Procédé selon l'une quelconque des revendications 1 à 12, dans lequel l'oligonucléotide comprend un ou plusieurs nucléosides choisis dans le groupe constitué par 2'-O-alkyl-ARN, 2'-O-méthyl-ARN, 2'-alcoxy-ARN, 2'-O-méthoxyéthyl-ARN (MOE), 2'-amino-ADN, 2'-fluoro-ARN, le nucléoside 2'-F-ANA et un ou plusieurs nucléosides LNA.

**14.** Procédé selon l'une quelconque des revendications 1 à 13, dans lequel l'oligonucléotide comprend des liaisons internucléosidiques phosphorothioate.

**15.** Procédé selon l'une quelconque des revendications 1 à 14, dans lequel le cation ionique du sel est choisi parmi un cation métallique, tel qu'un cation de sodium ou de potassium, ou un cation d'ammonium.

**16.** Procédé selon l'une quelconque des revendications 1 à 15, dans lequel l'oligonucléotide a une longueur de 8 à 25 nucléotides contigus, telle que de 12 à 20 nucléotides.

Figure 1

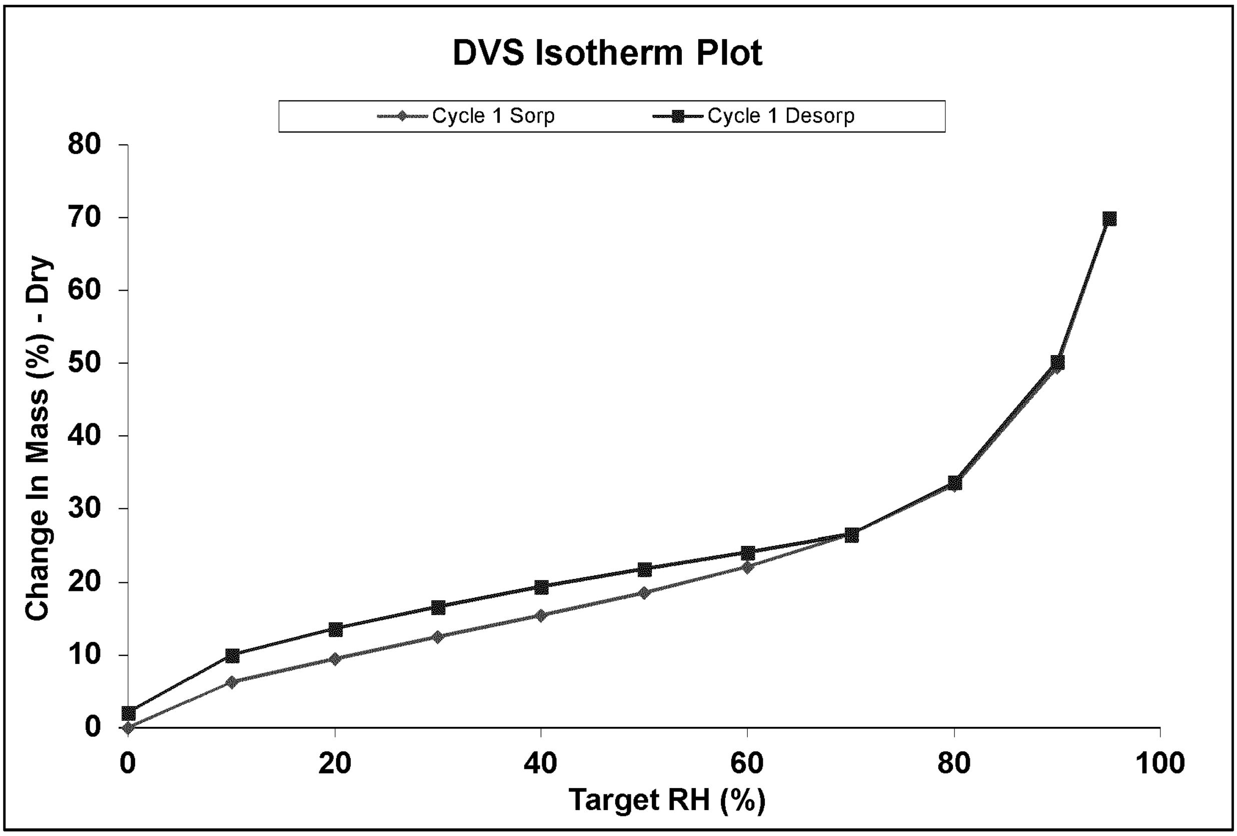
DVS Isotherm Plot
Cycle 1 Sorp
Cycle 1 Desorp
Change In Mass (%) - Dry
0
10
20
30
40
50
60
70
80
Target RH (%)
0
20
40
60
80
100

Figure 2

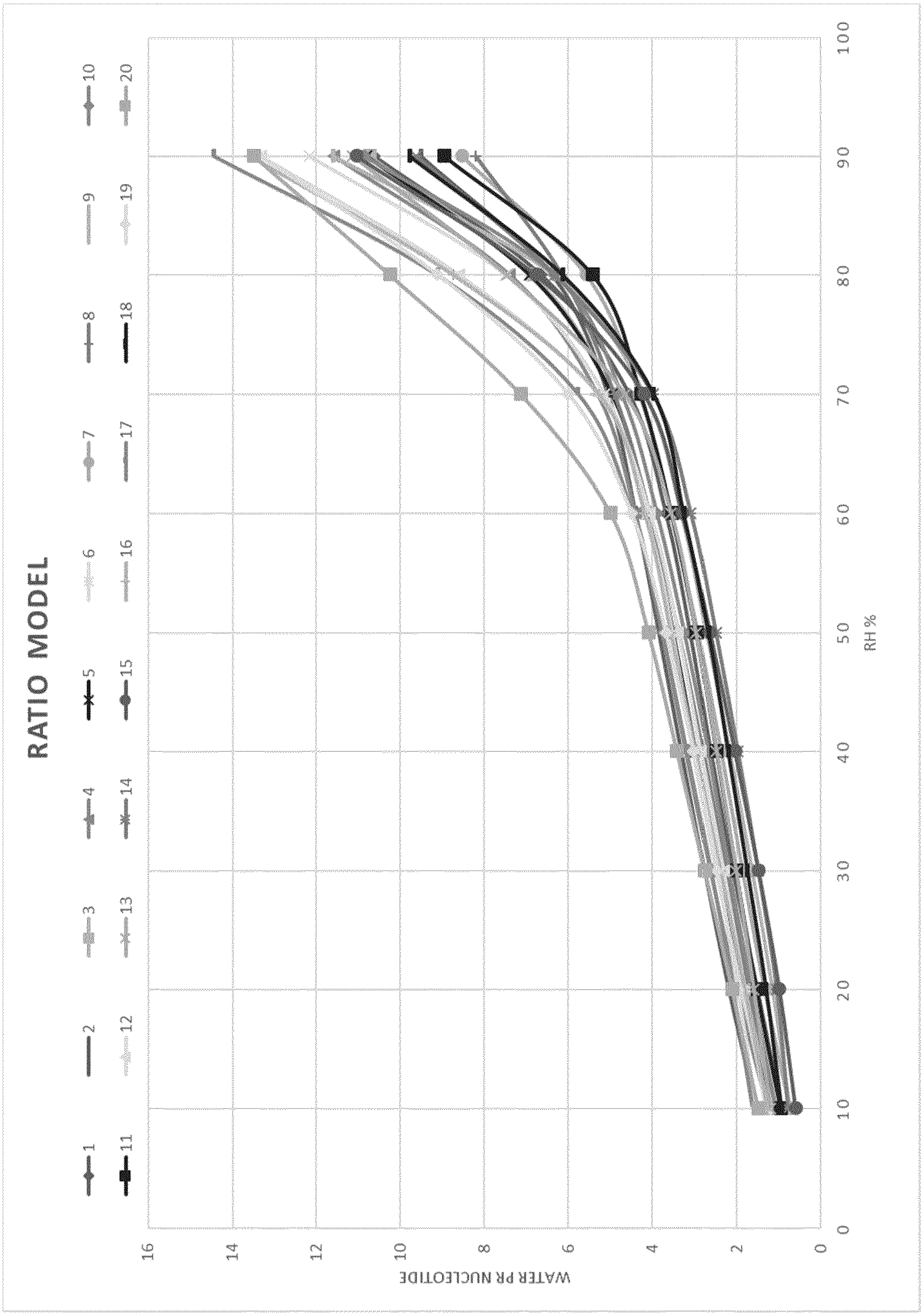
RATIO MODEL
1
2
3
4
5
6
7
8
9
10
11
12
13
14
15
16
17
18
19
20
RH %
WATER PR NUCLEOTIDE
0
10
20
30
40
50
60
70
80
90
100
0
2
4
6
8
10
12
14
16

Figure 3

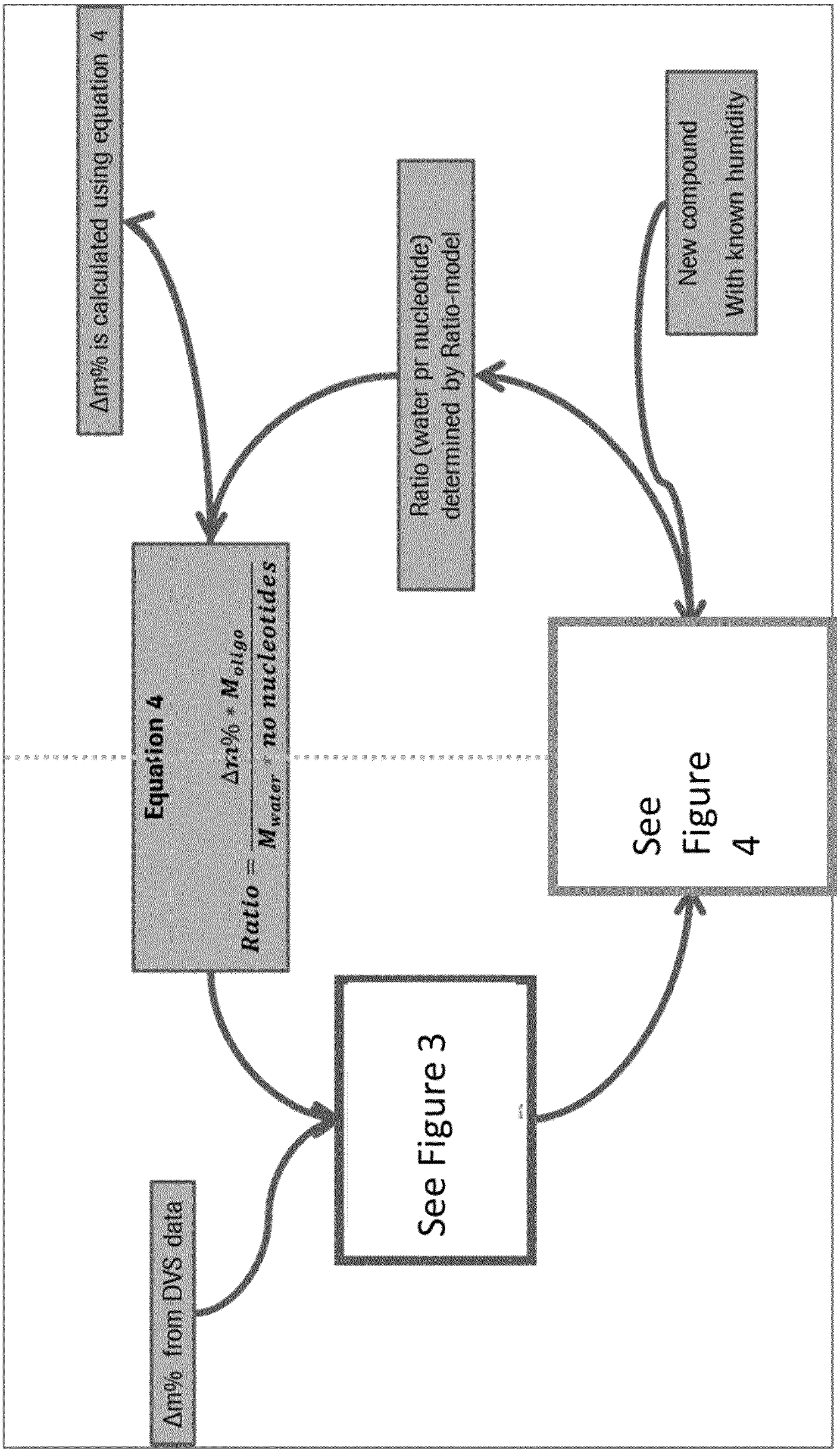
Δm% is calculated using equation 4
Ratio (water pr nucleotide) determined by Ratio-model
New compound With known humidity
Equation 4
$$Ratio = \frac{\Delta m\% * M_{oligo}}{M_{water} * no\ nucleotides}$$
See Figure 4
See Figure 3
Δm% from DVS data

Figure 4

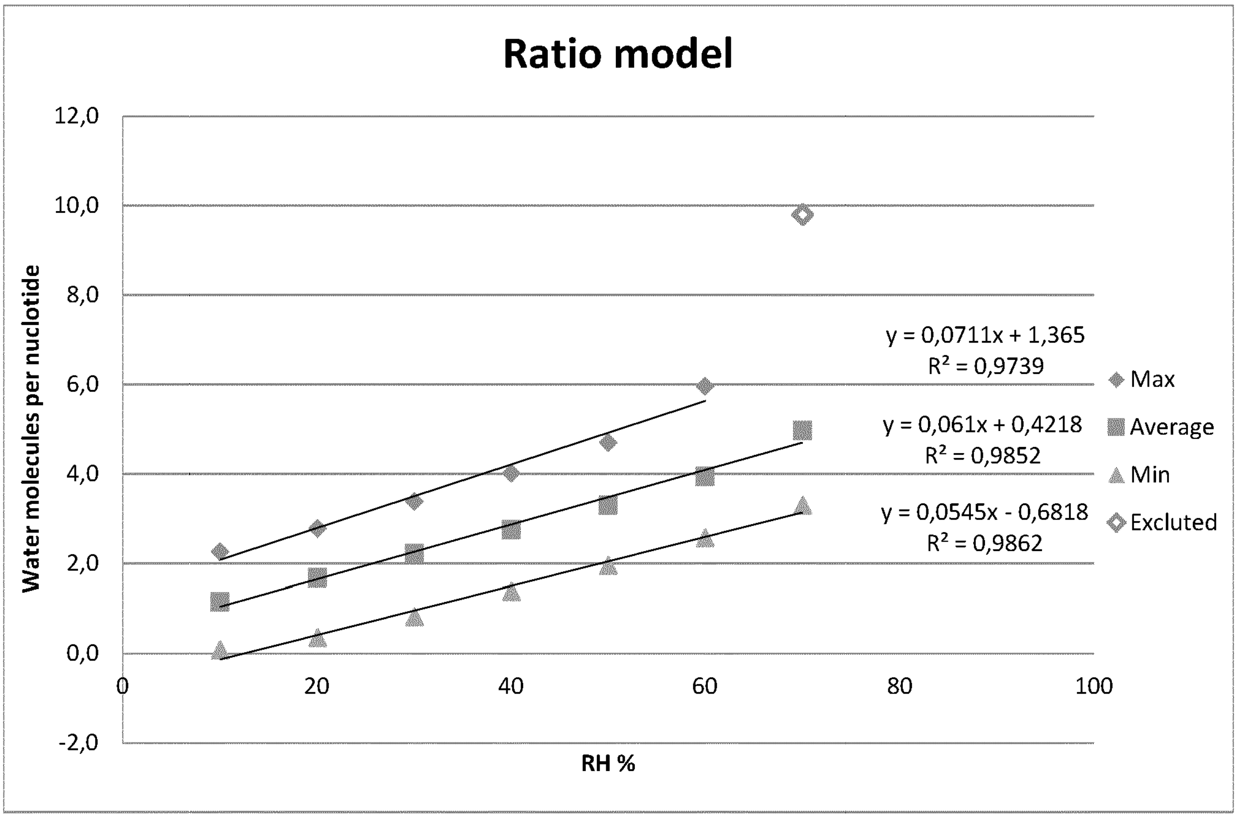
Ratio model
12,0
10,0
8,0
6,0
4,0
2,0
0,0
-2,0
0
20
40
60
80
100
Water molecules per nuclotide
RH %
y = 0,0711x + 1,365
R² = 0,9739
y = 0,061x + 0,4218
R² = 0,9852
y = 0,0545x - 0,6818
R² = 0,9862
Max
Average
Min
Excluted

**Figure 5**

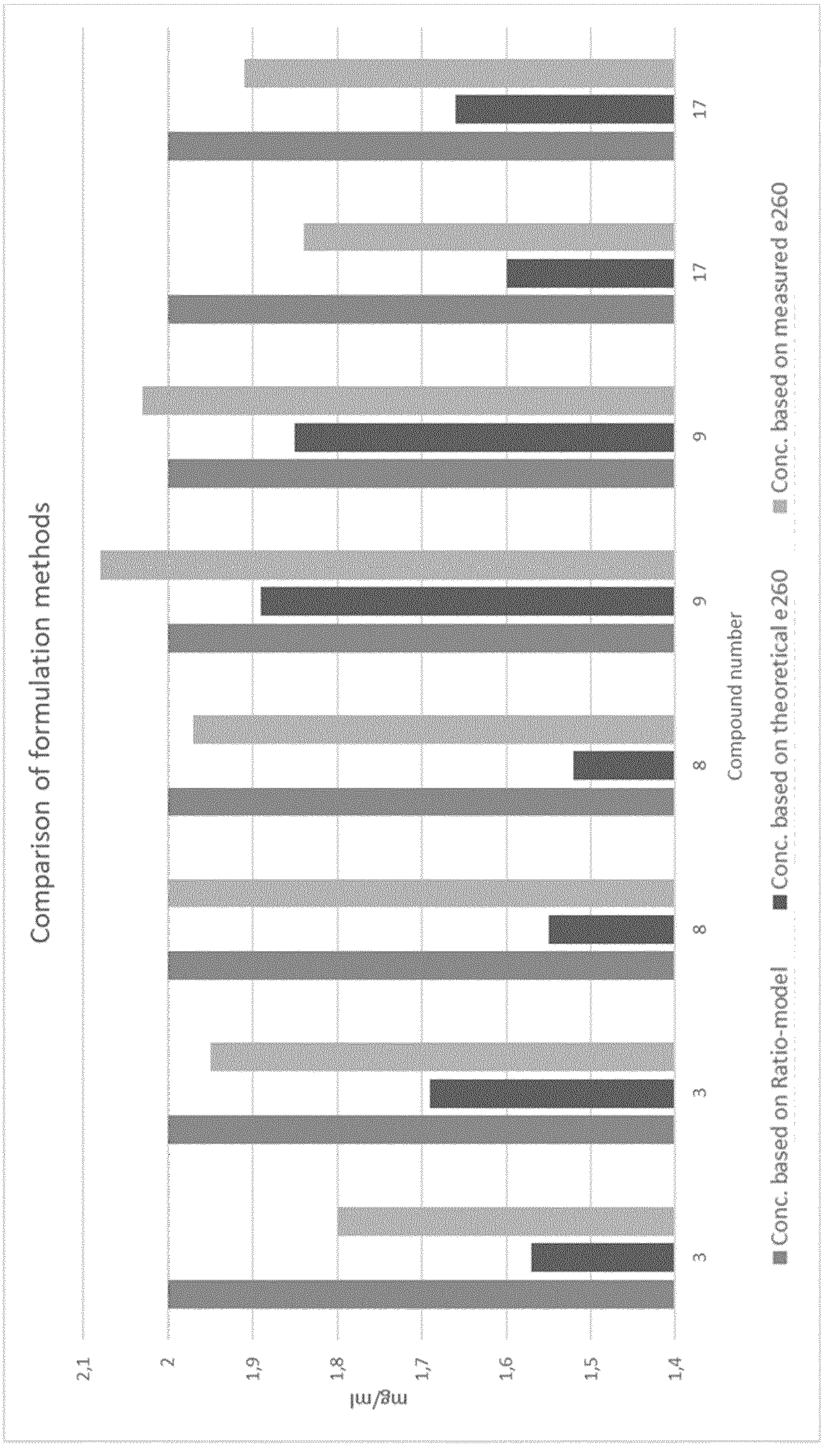
Comparison of formulation methods
mg/ml
2,1
2
1,9
1,8
1,7
1,6
1,5
1,4
3
3
8
8
9
9
17
17
Compound number
Conc. based on Ratio-model
Conc. based on theoretical e260
Conc. based on measured e260

Figure 6

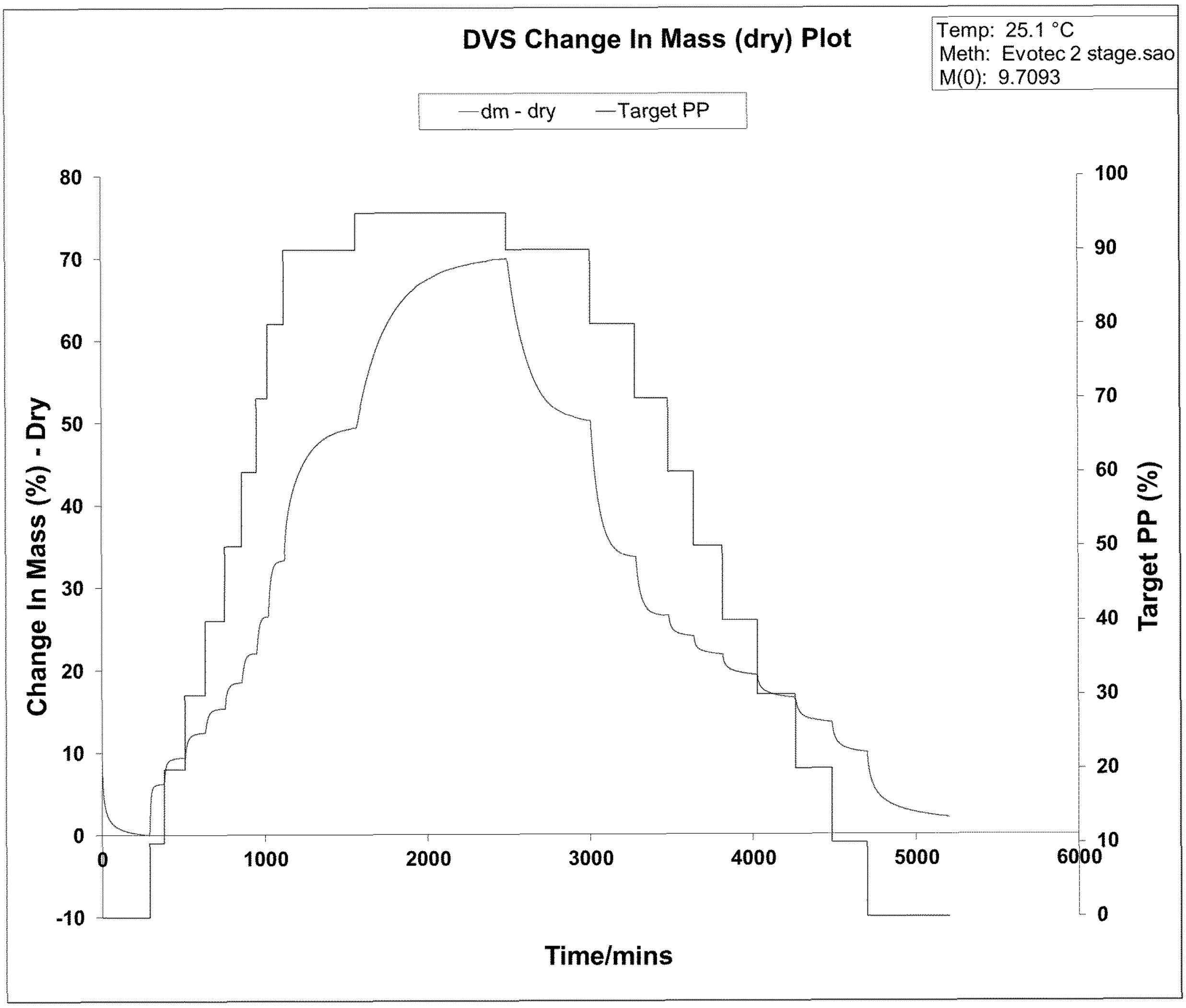
DVS Change In Mass (dry) Plot
Temp: 25.1 °C
Meth: Evotec 2 stage.sao
M(0): 9.7093
—dm - dry
—Target PP
Change In Mass (%) - Dry
Target PP (%)
Time/mins
80
70
60
50
40
30
20
10
0
-10
100
90
80
70
60
50
40
30
20
10
0
1000
2000
3000
4000
5000
6000

**Figure 7**

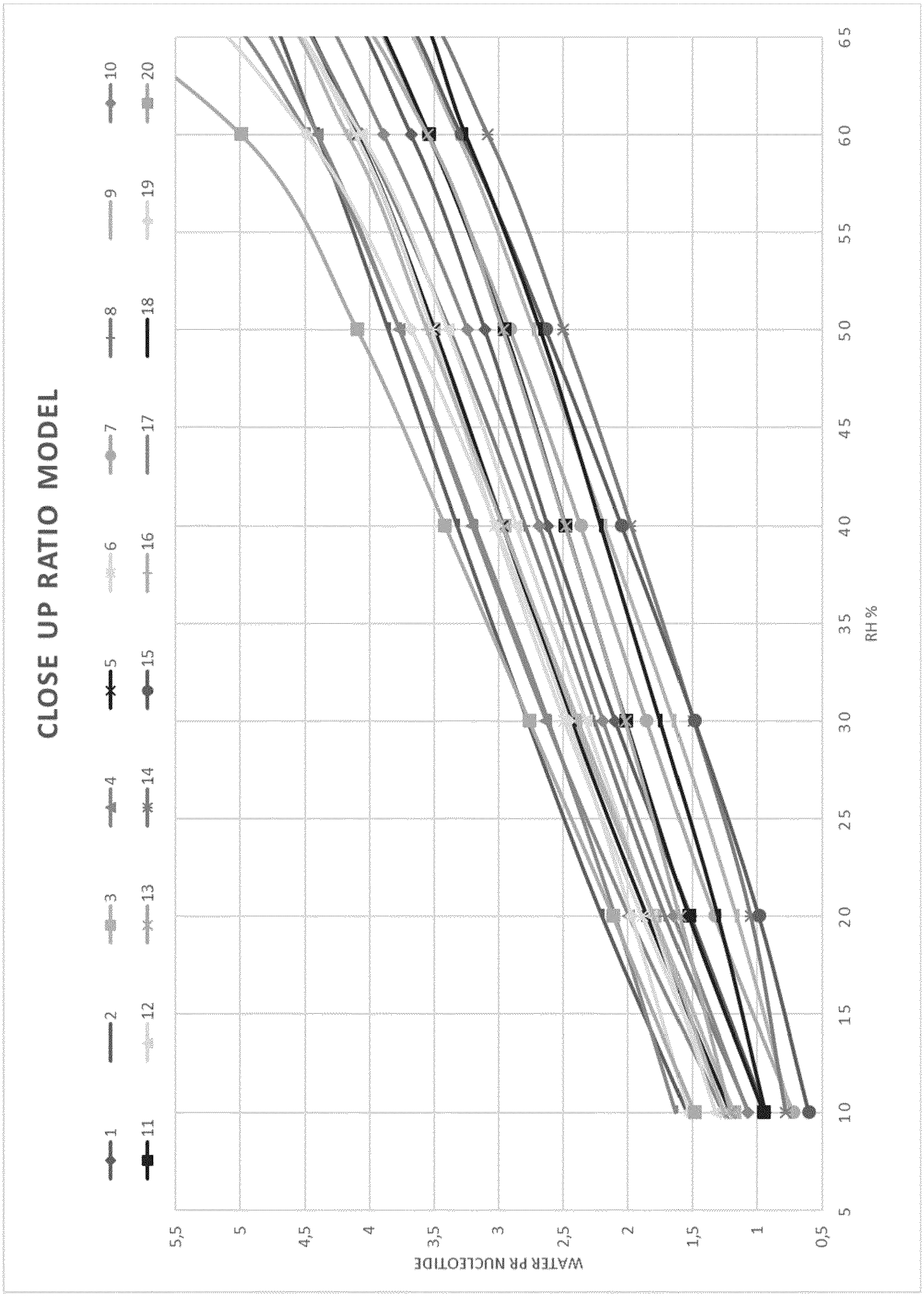
CLOSE UP RATIO MODEL
1
2
3
4
5
6
7
8
9
10
11
12
13
14
15
16
17
18
19
20
WATER PR NUCLEOTIDE
5,5
5
4,5
4
3,5
3
2,5
2
1,5
1
0,5
RH %
5
10
15
20
25
30
35
40
45
50
55
60
65

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2007009009 A
- WO 2011017521 A
- WO 2013154798 A
- WO 99014226 A
- WO 0066604 A
- WO 98039352 A
- WO 2004046160 A
- WO 00047599 A
- WO 2007134181 A
- WO 2010077578 A
- WO 2010036698 A
- WO 2007090071 A
- WO 2009006478 A
- WO 2011156202 A
- WO 2008154401 A
- WO 2009067647 A
- WO 2008150729 A
- WO 2013022984 A
- WO 2008049085 A
- WO 2012109395 A
- WO 2014076195 A
- WO 2015113922 A
- WO 2007112754 A
- WO 2014076196 A
- WO 2014207232 A
- WO 2014179620 A
- EP 1495769 A
- WO 9965925 A
- US 2012122801 A
- US 5885968 A
- WO 2016055601 A
- WO 2008034123 A
- WO 2014118267 A


### Non-patent literature cited in the description


- **VOVELLE ; GOODFELLOW.** *Int. J. Biol. Macromol.,* 1990, vol. 12, 369-373
- Handbook of Analysis of Oligonucleotide and Related Products. 286-289
- **MURPHY ; TRAPANE.** *Analytical Biochemistry,* 1996, vol. 240, 273-282
- **HIRAO et al.** *Accounts of Chemical Research,* 2012, vol. 45, 2055
- **BERGSTROM.** *Current Protocols in Nucleic Acid Chemistry,* 2009, (37
- **FREIER ; ALTMANN.** *Nucl. Acid Res.,* 1997, vol. 25, 4429-4443
- **UHLMANN.** *Curr. Opinion in Drug Development,* 2000, vol. 3 (2), 293-213
- **DELEAVEY ; DAMHA.** *Chemistry and Biology,* 2012, vol. 19, 937
- **MORITA et al.** *Bioorganic & Med.Chem. Lett.,* vol. 12, 73-76
- **SETH et al.** *J. Org. Chem.,* 2010, vol. 75 (5), 1569-81
- **MITSUOKA et al.** *Nucleic Acids Research,* 2009, vol. 37 (4), 1225-1238
- **WAN AND SETH.** *J. Medical Chemistry,* 2016, vol. 59, 9645-9667
- **MANOHARAN.** Antisense Drug Technology, Principles, Strategies, and Applications. Marcel Dekker, Inc, 2001
- **MANOHARAN.** *Antisense and Nucleic Acid Drug Development,* 2002, vol. 12, 103
- **PLATTS et al.** *J. Am. Chem. Soc.,* 1996, vol. 118 (11), 2726-2733
- **YANG et al.** *Bioconjug Chem,* 2009, vol. 20 (2), 213-21
- **ZATSEPIN ; ORETSKAYA.** *Chem Biodivers.,* 2004, vol. 1 (10), 1401-17
- **JOBST, S.T. ; DRICKAMER, K.** *JB.C.,* 1996, vol. 271, 6686
- **BIESSEN et al.** *J. Med. Chern.,* 1995, vol. 39, 1538-1546
- **KHOREV et al.** *Bioorg. Med. Chem.,* 2008, vol. 16, 5216
- **DUFF et al.** *Methods Enzymol,* 2000, vol. 313, 297
- **BIESSEN et al.** *Cardovasc. Med.,* 1999, vol. 214